# EUROPÄISCHE PATENTSCHRIFT

(11) **EP 2 370 177 B1**
(45) Veröffentlichungstag und Bekanntmachung des Hinweises auf die Patenterteilung: **15.11.2017**
(21) Anmeldenummer: 09760879.8
(22) Anmeldetag: 30.11.2009
(51) Int. Cl.: A61Q 7/02, A61K 8/60, A61K 8/44, A61K 8/49

(54) **NEUE KOSMETISCHE ZUSAMMENSETZUNGEN MIT HAARWUCHSINHIBIERENDER WIRKUNG**
NOVEL COSMETIC COMPOSITIONS HAVING HAIR GROWTH INHIBITING EFFECT
NOUVELLES COMPOSITIONS COSMÉTIQUES PERMETTANT D'INHIBER LA CROISSANCE PILEUSE

(30) Priorität: 01.12.2008 DE 102008059703
(43) Veröffentlichungstag der Anmeldung: 05.10.2011
(73) Patentinhaber: Henkel AG & Co. KGaA, 40589 Düsseldorf (DE)
(72) Erfinder: KROPF, Christian, 40724 Hilden (DE); HUCHEL, Ursula, 50670 Köln (DE); PETERSOHN, Dirk, 50996 Köln (DE); COX, Bruce, Scottsdale AZ 85259 (US); WELß, Thomas, 40591 Düsseldorf (DE)
(86) Internationale Anmeldenummer: PCT/EP2009/066048
(87) Internationale Veröffentlichungsnummer: WO 2010/063673

(56) Entgegenhaltungen:
- WO-A2-03/089601
- WO-A2-2005/051288
- WO-A2-2009/007257
- DE-A1- 2 364 525
- DE-A1- 10 041 482
- DE-A1- 10 160 966
- US-B1- 6 218 435
- Thomas Henle: "Efficient determination of individual Maillard compounds in heat-treated milk products by amino-acid analysis", International Dairy Journal, 1. Januar 1991 (1991-01-01), Seiten 125-135, XP55033133, Gefunden im Internet: URL:http://ac.els-cdn.com/095869469190004R /1-s2.0-095869469190004R-main.pdf?_tid=30f e52ac5998dc46e724b2727772361a&acdnat=13426 13696_06bfeec61cc13a0f9ed00260d9e170fa [gefunden am 2012-07-18]
- JOSÉ A.B BAPTISTA ET AL: "Indirect determination of Amadori compounds in milk-based products by HPLC/ELSD/UV as an index of protein deterioration", FOOD RESEARCH INTERNATIONAL, Bd. 37, Nr. 8, 1. Januar 2004 (2004-01-01) , Seiten 739-747, XP55033131, ISSN: 0963-9969, DOI: 10.1016/j.foodres.2004.02.006
- Franz Ledl: "Formation of maltosine, a product of the Maillard reaction with a pyridone structure", Zeitschrift für Lebensmittel-Untersuchung und -Forschung, 1. Januar 1989 (1989-01-01), Seiten 207-211, XP55033149, Gefunden im Internet: URL:http://www.springerlink.com/content/r6 k1306376q12564/fulltext.pdf [gefunden am 2012-07-18]

## Beschreibung

Die vorliegende Erfindung betrifft kosmetische und dermatologische Zusammensetzungen, die eine verbesserte Wirkung gegen Haarwuchs aufweisen.

Aus modischen, ästhetischen oder anderen Gründen ist Körperbehaarung oft unerwünscht. Von besonderem Interesse sind daher kosmetische Wirkstoffe zur Behandlung der Haut, die eine Haarwuchs inhibierende Wirkung aufweisen. Es besteht daher nach wie vor das Bedürfnis, den Stand der Technik durch neue den Haarwuchs inhibierende Wirkstoffe zu bereichern.

Die haarwuchsreduzierende Wirkung von 3-Deoxyglucose wird in der Patentanmeldung US 6,218,435 B1 beschrieben.

Die Offenlegungsschrift DE 10041482 offenbart so genannte AGEs (Advanced Glycation Endproducts) als kosmetische Wirkstoffe zur Induktion und Intensivierung von Bräunungsmechanismen der menschlichen Haut und die Verwendung einer oder mehrerer Substanzen aus der Gruppe der AGEs und/oder deren Vorläufern zur Steigerung der Melaninsynthese der Haut.

Die Offenlegungsschrift DE 10160966 offenbart die Lehre zur Bereitstellung eines Haarfärbemiittels, enthaltend mindestens eine Verbindung aus der Gruppe der AGEs, deren synthetischen Vorstufen und/oder der Lipofuscine und die Verwendung eines solchen Haarfärbemittemittels zur Steigerung der Melaninsynthese in der Haarwurzel und der Einlagerung von Melanin im Haar. Beiden Schriften ist gemeinsam, dass kosmetische Wirkstoffe aus der Gruppe der AGEs zur Steigerung der Melaninsynthese im Haar und/oder der Haut benutzt werden. Ein Hinweis auf andere oder weitergehende biologisch relevante Wirkungen der AGEs sind den beiden letztgenannten Dokumenten nicht zu entnehmen.

Überraschenderweise wurde nun gefunden, dass Verbindungen aus der Gruppe der AGEs und/oder deren Vorläufern auch als haarwuchsinhibierender Wirkstoff verwendet werden können. Gegenstand der vorliegenden Anmeldung ist daher die Verwendung einer kosmetischen oder dermatologischen Zusammensetzung, die in einem kosmetischen oder dermatologischen Träger mindestens eine Verbindung aus der Gruppe der Advanced Glycation Endproducts (AGEs) und/oder deren Vorläufern enthalten, ausgewählt aus den Reaktionsprodukten der Umsetzung mindestens einer Verbindung aus der Gruppe der Zucker, bevorzugt ausgewählt aus den Aldosen, vorzugsweise ausgewählt aus Glucose, Laktose, Galactose und/oder Mannose, mit mindestens einer Verbindung aus der Gruppe der Aminosäuren, bevorzugt ausgewählt aus Lysin, Hydroxylysin, Arginin, Tryptophan und/oder Histidin, dadurch gekennzeichnet, dass sie mindestens eine Verbindung gemäß Formel 15 oder 15a enthält, zur topischen Behandlung der Haut.

Erfindungsgemäß sind unter dem allgemein verwendeten Begriff "Haut" die Haut selbst, die Schleimhaut sowie die Hautanhangsgebilde, sofern sie lebende Zellen umfassen, insbesondere Haarfollikel, Haarwurzel, Haarzwiebel, das ventrale Epithel des Nagelbetts (Lectulus) sowie Talgdrüsen und Schweißdrüsen, zu verstehen. Bevorzugt im Sinne der Erfindung ist unter dem allgemein verwendeten Begriff "Haut" die menschliche Haut zu verstehen.

Die Bezeichnung AGE kommt vom englischen Begriff "Advanced Glycation Endproducts". AGEs im Sinne der vorliegenden Erfindung sind beispielsweise erhältlich gemäß der folgenden (beispielhaften) Reaktionsgleichung:

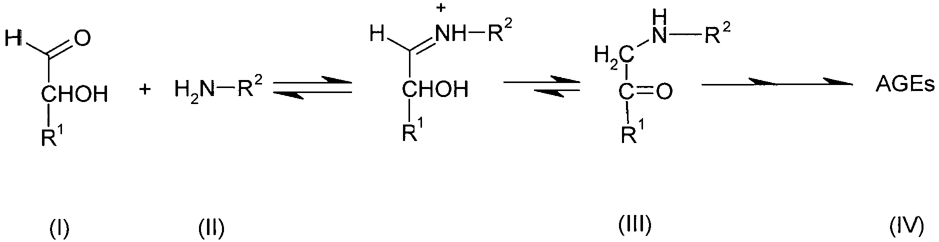

Vorteilhaft im Sinne der vorliegenden Erfindung sind neben den eigentlichen AGEs auch deren Vorläufer, wie z.B. Amadori- oder Maillard-Produkte (entsprechend (III) in obiger Reaktionsgleichung) bzw. so genannte EGEs (Early Glycation Endproducts), die beispielsweise als Zwischenprodukte bei der Umlagerung von den Amadoriprodukten (III) zu den AGEs (IV) erhältlich sind.

Weitere Produkte der Maillard-Reaktion, insbesondere solche, die sich auch vom Amadoriprodukt ableiten, wie insbesondere Maltol (Larixin, 3-Hydroxy-2-methyl-4H-pyran-4-on) und 4-Hydroxy-2,5-dimethyl-3(2H)-furanon (Furaneol ®), sind ebenfalls vom Begriff "Vorläufer" mit umfasst.

Unter dem Begriff "Vorläufer" sind im Sinne der vorliegenden Erfindung ausdrücklich nicht die jeweiligen Ausgangsstoffe der Reaktion, also die nicht umgesetzten Edukte zu verstehen.

Vorteilhafte Ausgangsstoffe (I) tragen eine Aldehydgruppe (CHO) und werden beispielsweise aus der Gruppe der Zucker (Aldosen, z. B. Triosen, Tetrosen, Pentosen, Hexosen und dergleichen wie Glucose, Galactose, Mannose usw.) gewählt. Vorteilhafte Ausgangsstoffe (II) weisen eine freie Aminogruppe auf und werden beispielsweise aus der Gruppe der Aminosäuren und der Gruppe der Peptide mit endständigen Aminogruppen gewählt, insbesondere Lysin, Hydroxylysin, Arginin, Tryptophan und Histidin sind vorteilhaft. Ferner vorteilhaft kann die freie Aminogruppe auch aus N-terminalen Enden von Proteinen und Peptiden stammen, ferner auch vorteilhaft Sphingosin sowie Dihydrosphingosin und deren Homologen mit verschieden langen (ungesättigten) Acylresten. Bevorzugt enthalten erfindungsgemäße AGEs mindestens einen stickstoffhaltigen und/oder sauerstoffhaltigen Fünf- und/oder Sechsring.

Die dargestellten Strukturformeln sollen die Erfindung selbstverständlich nicht auf bestimmte Isomere der erfindungsgemäßen Substanzen beschränken. Vielmehr sind auch nicht dargestellte Isomere bzw. Isomerengemische vorteilhaft im Sinne der vorliegenden Erfindung.

Die durch Umsetzung mindestens einer Verbindung aus der Gruppe der Zucker mit mindestens einer Verbindung aus der Gruppe der Aminosäuren dargestellten Advanced Glycation Endproducts (AGEs) und/oder deren Vorläufern sind sind besondere dann bevorzugt, wenn das molare Verhältnis von Zucker zu Aminosäure bei der Umsetzung zwischen 10:1 und 1:1, besonders bevorzugt zwischen 7:1 und 1:1 und insbesondere bei 5:1 oder 4:1 liegt.

Weitere bevorzugte Advanced Glycation Endproducts (AGEs) und/oder deren Vorläufer werden durch die Umsetzung einer Aminosäure ausgewählt aus Lysin, Hydroxylysin, Arginin, Tryptophan und/oder Histidin erhalten.

Besonders bevorzugt sind Lysin- und/oder Arginin-basierte Advanced Glycation Endproducts (AGEs) und/oder deren Vorläufer, die durch Umsetzung von Zuckern, vorzugsweise Glukose, Laktose, Galactose und/oder Mannose, insbesondere Glukose und/oder Laktose mit den Aminosäuren Lysin und/oder Arginin, vorzugsweise bei Reaktionstemperaturen von 15°C bis 95°C gebildet werden. Besonders bevorzugt sind die Umsetzungsprodukte, die bei Reaktionen in Temperaturbereichen zwischen 16°C und 35°C, zwischen 36°C und 50°C, zwischen 51°C und 75°C oder zwischen 76°C und 95°C, insbesondere bei 15°C, 20°C, 25°C, 30°C, 35°C, 40°C, 45°C, 50°C, 55°C, 60°C, 65°C, 70°C, 75°C, 80°C, 85°C, 90°C oder 95°C gebildet werden.

Weiterhin bevorzugt sind Lysin- und/oder Arginin-Advanced Glycation Endproducts (AGEs) und/oder deren Vorläufer, die durch Umsetzung von Zuckern, vorzugsweise Glukose, Laktose, Galactose und/oder Mannose, insbesondere Glukose und/oder Laktose mit den Aminosäuren Lysin und/oder Arginin bei Reaktionszeiten von 0,5 h bis 48 h gebildet werden.

Maltosin 15 sowie Pyridosin 15a sind erfindungsgemäße Lysin-basierte AGEs und/oder deren Vorläufer und Produkte der Maillard-Reaktion von Lysin A mit Zuckern.

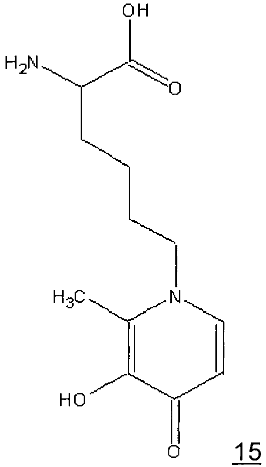

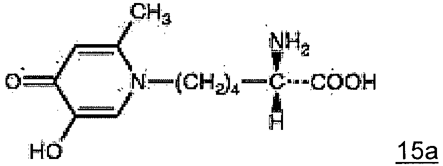

Eine bevorzugte Ausführungsform der Erfindung ist die Verwendung einer erfindungsgemäßen kosmetischen Zusammensetzung mit einem Anteil einer Verbindung oder einer Mischung verschiedener Verbindungen aus der Gruppe AGEs und/oder deren Vorläufern von 0,00001 bis 15 Gew.-%, vorzugsweise von 0,0001 bis 5 Gew.-%, besonders bevorzugt von 0,001 bis 1 Gew.-%, jeweils bezogen auf die Gesamtzusammensetzung.

Besonders bevorzugte erfindungsgemäße kosmetische oder dermatologische Zusammensetzungen enthalten - bezogen auf das Gewicht der anwendungsbereiten Zusammensetzung - 0,00001 bis 15 Gew.-%, vorzugsweise 0,0001 bis 10 Gew.-%, besonders bevorzugt 0,001 bis 5 Gew.-%, weiter bevorzugt 0,01 bis 4 Gew.-% und insbesondere 0,1 bis 2 Gew.-% Advanced Glycation Endproducts (AGEs) und/oder deren Vorläufer.

Ein besonders bevorzugtes AGE ist Maltosin, das besonders vorteilhaft in erfindungsgemäßen Zusammensetzungen enthalten ist. Erfindungsgemäß äußerst bevorzugte kosmetische oder dermatologische Zusammensetzungen enthalten - bezogen auf das Gewicht der anwendungsbereiten Zusammensetzung - 0,0001 bis 15 Gew.-%, vorzugsweise 0,001 bis 12,5 Gew.-%, besonders bevorzugt 0,01 bis 10 Gew.-%, weiter bevorzugt 0,1 bis 5 Gew.-% und insbesondere 0,25 bis 2,5 Gew.-% Maltosin (Formeln 15/15a).

Erfindungsgemäß bevorzugte Zusammensetzungen sind dadurch gekennzeichnet, dass sie weiterhin Purin und/oder mindestens ein Derivat des Purins enthalten. Purin (7*H*-Imidazo[4,5-*d*] pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten. Purin kann beispielsweise aus Aminoacetonitril und Formamid hergestellt werden. Purine und Purinderivate werden oft aus Naturstoffen isoliert, sind aber auch auf vielen Wegen synthetisch zugänglich. Purine und Purinderivate können die Haarwuchs inhibierende Wirkung der AGEs vorteilhaft unterstützen.

Bevorzugte erfindungsgemäße Zusammensetzungen enthalten Purin und/oder Purinderivat(e) in engeren Mengenbereichen. Hier sind erfindungsgemäß bevorzugte kosmetische Zusammensetzungen dadurch gekennzeichnet, dass sie - bezogen auf ihr Gewicht - eine Gesamtmenge von 0,001 - 2,5 Gew.-%, bevorzugt 0,01 - 1 Gew.-%, besonders bevorzugt 0,1 - 0,8 Gew.-% und insbesondere 0,2 - 0,5 Gew.-% Purin(e) und/oder Purinderivat(e) enthalten.

Unter Purin, den Purinen und den Purinderivaten sind einige Vertreter erfindungsgemäß besonders bevorzugt. Erfindungsgemäß bevorzugte kosmetische Zusammensetzungen sind dadurch gekennzeichnet, dass sie Purin und/oder Purinderivat(e) der Formel (PUR-I) enthalten, 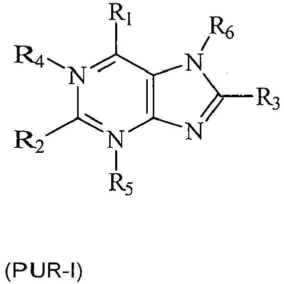 in der die Reste R¹, R² und R³ unabhängig voneinander ausgewählt sind aus -H, - OH, -NH₂ und - SH und die Reste R⁴, R⁵ und R⁶ unabhängig voneinander ausgewählt sind aus -H, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe.

Erfindungsgemäß besonders bevorzugte kosmetische Zusammensetzungen sind dadurch gekennzeichnet, dass sie Purin und/oder Purinderivat(e) der Formel (PUR-I) enthalten, in der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, -CH₃ und -CH₂-CH₃, wobei folgende Verbindungen außerordentlich bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).

Verbindungen der allgemeinen Formel (PUR-I), in denen R¹ und/oder R² einer OH-Gruppe entsprechen, existieren in tautomeren Verbindungen, insbesondere solchen der allgemeinen Formel (PUR-II), ausgehend von (PUR-I) mit R¹ = R² = OH : 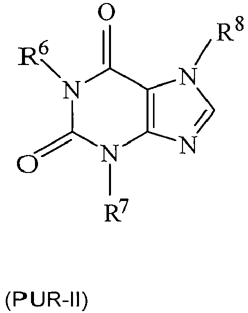 wobei in der Formel (PUR-II) die Reste R⁶, R⁷ und R⁸, die gleich oder verschieden sein können, bevorzugt ausgewählt sind aus einem Wasserstoffatom, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe, wobei Coffein (R⁶ = R⁷ = R⁸ = CH₃), Theobromin (R⁶ = H, R⁷ = R⁸ = CH₃) und Theophyllin (R⁶ = R⁷ = CH₃, R⁸ = H) außerordentlich bevorzugt sind.

Besonders bevorzugt sind Zusammensetzungen, die Maltosin und Coffein enthalten. Weiterhin besonders bevorzugt sind Zusammensetzungen, die Maltosin und Theobromin enthalten. Weiterhin besonders bevorzugt sind Zusammensetzungen, die Maltosin und Theophyllin enthalten.

Je nach gewünschtem Anwendungszweck der kosmetischen Zusammensetzung kann dabei die Art und Menge des Purinderivates variieren. In Körperpflegemitteln zur gleichzeitigen haarwuchsinhibierenden und Anticellulite-Behandlung der Haut, hat sich insbesondere Coffein bewährt, das in Anticellulite-Mitteln, insbesondere Cremes, Gelen und Lotionen, vorzugsweise in Mengen von 0,01 - 2 Gew.-%, weiter bevorzugt 0,1 - 1,5 Gew.-% und besonders bevorzugt 0,2 - 0,8 Gew.-%, jeweils bezogen auf das Mittel, enthalten ist.

Weitere bevorzugte erfindungsgemäße Zusammensetzungen sind dadurch gekennzeichnet, dass sie zusätzlich zu dem mindestens einen Advanced Glycation Endproduct (AGEs) und/oder dessen Vorläufer als weiteren wesentlichen Inhaltsstoff mindestens eine natürliche Betainverbindung enthalten. Natürliche Betainverbindungen können die Haarwuchs inhibierende Wirkung der AGEs vorteilhaft unterstützen. Erfindungsgemäß eingesetzte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen.

Erfindungsgemäß bevorzugte Betainverbindungen sind Betain (Me₃N⁺-CH₂-COO-) und Carnitin (Me₃N⁺-CH₂-CHOH-CH₂-COO⁻), jeweils mit Me = Methyl.

Bevorzugte erfindungsgemäße Zusammensetzungen sind dadurch gekennzeichnet, dass sie mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte Zusammensetzung, enthalten.

Weitere bevorzugte erfindungsgemäße Zusammensetzungen sind dadurch gekennzeichnet, dass sie zusätzlich zu dem mindestens einen Advanced Glycation Endproduct (AGEs) und/oder dessen Vorläufer als weiteren wesentlichen Inhaltsstoff mindestens eine Substanz enthalten, die ausgewählt ist aus Harnstoff und alkyl- oder hydroxyalkylsubstituierten Harnstoffen. Erfindungsgemäß bevorzugte alkyl- oder hydroxyalkylsubstituierte Harnstoffverbindungen weisen die allgemeine Formel (UREA-I) auf, 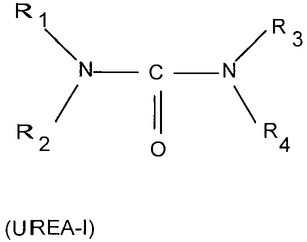 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Alkyl- oder hydroxyalkylsubstituierte Harnstoffverbindungen der allgemeinen Formel (UREA-I) können die Haarwuchs inhibierende Wirkung der AGEs vorteilhaft unterstützen.

Erfindungsgemäß besonders bevorzugte alkyl- oder hydroxyalkylsubstituierte Harnstoffverbindungen der allgemeinen Formel (UREA-I) sind ausgewählt aus Verbindungen, bei denen jeweils mindestens einer der Reste R₁ und R² bzw. R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Besonders bevorzugte C₂-C₆-Hydroxyalkyl-Gruppen, die mit 1 bis 5 Hydroxylgruppen oder C₁-C₄-Hydroxyalkyl-Gruppen substituiert sind, sind ausgewählt aus 2-Hydroxyethyl-, 3-Hydroxypropyl-, 2-Hydroxypropyl, 2-Hydroxyisopropyl- und 4-Hydroxybutyl-Gruppen, wobei die 2-Hydroxyethyl-Gruppe außerordentlich bevorzugt ist. Ebenfalls außerordentlich bevorzugt ist Bis-N,N'-(2-Hydroxyethyl)-harnstoff. Harnstoff selbst ist ebenfalls besonders bevorzugt. Weiterhin bevorzugt ist es, Mischungen von Harnstoff und alkyl- oder hydroxyalkylsubstituierten Harnstoffverbindungen einzusetzen. Außerordentlich bevorzugt sind Mischungen von Harnstoff und Bis-N,N'-(2-Hydroxyethyl)-harnstoff. Besonders bevorzugt sind Zusammensetzungen, die Maltosin und Bis-N,N'-(2-Hydroxyethyl)-harnstoff enthalten. Weiterhin besonders bevorzugt sind Zusammensetzungen, die Maltosin, Bis-N,N'-(2-Hydroxyethyl)-harnstoff und Harnstoff enthalten.

Weitere bevorzugte erfindungsgemäße Zusammensetzungen sind dadurch gekennzeichnet, dass sie zusätzlich zu dem mindestens einen Advanced Glycation Endproduct (AGEs) und/oder dessen Vorläufer als weiteren wesentlichen Inhaltsstoff mindestens eine Substanz enthalten, die ausgewählt ist aus Monomeren, Oligomeren und Polymeren von Aminosäuren, N-C₂-C₂₄-Acyl-aminosäuren und/oder den Estern und/oder den physiologisch verträglichen Salzen dieser Substanzen, sowie Mischungen dieser Wirkstoffe.

Die Monomere der Aminosäuren und/oder der N-C₂-C₂₄-Acylaminosäuren sind bevorzugt ausgewählt aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Zink- und Natriumpyroglutamat und Natriumlauroylglutamat.

Der C₂ - C₂₄-Acylrest, mit dem die genannten Aminosäuren an der Aminogruppe derivatisiert sind, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.

Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.

Die physiologisch verträglichen Salze der erfindungsgemäß bevorzugten Aminosäuren oder Aminosäurederivate sind bevorzugt ausgewählt aus den Ammonium-, Alkalimetall-, Magnesium-, Calcium-, Aluminium-, Zink- und Mangan-Salzen. Besonders bevorzugt sind die Natrium-, Kalium-, Magnesium-, Zink- und Mangan-Salze; außerordentlich bevorzugt sind die Natrium-, Kalium- und Magnesiumsalze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/ oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-ß (*tissue growth factor*), der die Synthese von Collagen in dermalen Fibroblasten induziert, freizusetzen. Derartige Aminosäureoligomere werden im Stand der Technik als Wirkstoffe gegen die Hautalterung verwendet. Ihr Einsatz in Kombination mit einem Advanced Glycation Endproduct (AGEs) und/oder dessen Vorläufer als haarwuchsinhibierendes Hautbehandlungsmittel ist bislang nicht bekannt.

Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-ß-Ala-His, N-Acetyl-Tyr-Arg-hexyldecylester (z. B. Calmosensine von Sederma), Carnosin (ß-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN®-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn, Gly-Asp-Ser, N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im Folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.

Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).

Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.

Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.

Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt. Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Glu-Glu-Met-Gln-Arg-Arg, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (enthalten in dem Rohstoff Glistin von Exsymol).

Ein erfindungsgemäß besonders bevorzugter Aminosäureoligomer-Wirkstoff ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.

Ein weiterer erfindungsgemäß bevorzugter Wirkstoff, deren Gegenwart eine unerwartete Wirkungssteigerung der Advanced Glycation Endproducts (AGEs) und/oder deren Vorläufer bewirkt, ist ausgewählt aus Polymeren der Aminosäuren und/oder der N-C₂-C₂₄-Acylaminosäuren. Erfindungsgemäß kombinierte Polymere der-Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Besonders bevorzugt sind Sojaproteinhydrolysate, besonders bevorzugt Sojaproteinhydrolysate mit einer mittleren Molmasse (Mw) im Bereich von 1200 - 1800 Dalton, bevorzugt im Bereich von 1400 - 1700 Dalton, z. B. unter dem Namen Ridulisse C® von der Firma Silab erhältlich, und Sojaproteinhydrolysate mit einer mittleren Molmasse (Mw) im Bereich von 600 - 1000 Dalton, bevorzugt 800 Dalton, z. B. unter dem Namen Phytokine® von Coletica erhältlich. Der Proteinhydrolysat-Anteil in Ridulisse C enthält vier Molmassenfraktionen: 0,8 Gew.-% mit einer Mw > 12.500 Da, 22,2 Gew.-% mit 1355 Da < Mw < 12.500 Da, 43,1 Gew.-% 75 Da < Mw < 1355 Da und 33,9 Gew.-% < 75 Da.

Weiterhin erfindungsgemäß bevorzugt sind Keratinhydrolysate, insbesondere Wollkeratinhydrolysate. Weiterhin erfindungsgemäß bevorzugt sind Conchiolinhydrolysate, insbesondere solche, die unter den Bezeichnungen Pearl Protein Extract und Pearl Protein Extract BG von der Firma Maruzen erhältlich sind. Proteinhydrolysate können naturgemäß auch monomere Aminosäuren und Oligopeptide enthalten; ihre Zusammensetzung ist normalerweise nicht definiert. Ebenfalls bevorzugt ist der Einsatz von Acylderivaten der Proteinhydrolysate, z. B. in Form ihrer Fettsäure-Kondensationsprodukte.

Erfindungsgemäß bevorzugt sind auch kationisierte Proteinhydrolysate. Besonders bevorzugt sind kationische Proteinhydrolysate, deren zugrunde liegender Proteinanteil eine Molmasse von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein.

In einer weiteren bevorzugten Ausführungsform sind die erfindungsgemäß kombinierten Polymeren der Aminosäuren ausgewählt aus DNA-Reparaturenzymen. Erfindungsgemäß bevorzugte DNA-Reparaturenzyme sind Photolyase und T4 Endonuclease V. Unter DNA-Reparatur ist definitionsgemäß die Spaltung bzw. Entfernung von UV-induzierten Pyrimidindimeren aus der DNA zu verstehen.

Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.

Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.

Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.

Erfindungsgemäß besonders bevorzugte Zusammensetzungen sind dadurch gekennzeichnet, dass sie neben dem mindestens einen Advanced Glycation Endproduct (AGEs) und/oder dessen Vorläufer mindestens eines der Handelsprodukte Photosomes™ oder Ultrasomes™ in Gesamtmengen von 0,1 - 10 Gew.-%, bevorzugt 0,5 - 5,0 Gew.-% und besonders bevorzugt 1,0 - 4,0 Gew.-%, bezogen auf die gesamte erfindungsgemäße Zusammensetzung, enthalten.

Erfindungsgemäß besonders bevorzugte kosmetische oder dermatologische Zusammensetzungen sind dadurch gekennzeichnet, dass sie Maltosin und Photolyase enthalten. Weitere erfindungsgemäß besonders bevorzugte kosmetische oder dermatologische Zusammensetzungen sind dadurch gekennzeichnet, dass sie Maltosin und T4 Endonuclease V enthalten.

Erfindungsgemäß besonders bevorzugte kosmetische oder dermatologische Zusammensetzungen sind dadurch gekennzeichnet, dass sie neben dem mindestens einen Advanced Glycation Endproduct (AGEs) und/oder dessen Vorläufer mindestens einen kosmetischen Wirkstoff, der ausgewählt ist aus Monomeren, Oligomeren und Polymeren von Aminosäuren, N-C₂-C₂₄-Acyl-aminosäuren und/oder den Estern und/oder den physiologisch verträglichen Salzen dieser Substanzen, in einer Gesamtmenge von 0,00001 - 2 Gew.-%, bevorzugt 0,0001 - 1 Gew.-%, besonders bevorzugt 0,001 - 0,1 Gew.-% und außerordentlich bevorzugt 0,01 - 0,05 Gew.-%, jeweils bezogen auf den Gehalt an Aktivsubstanz in der gesamten Zusammensetzung, enthalten. Ein weiterer erfindungsgemäß bevorzugter Wirkstoff, dessen Gegenwart eine unerwartete Wirkungssteigerung des mindestens einen Advanced Glycation Endproduct (AGEs) und/oder dessen Vorläufer bewirkt, ist ausgewählt aus Polysacchariden. Erfindungsgemäß bevorzugte Polysaccharide sind solche, die mindestens einen Desoxyzucker-Baustein, insbesondere Fucose und/oder Rhamnose, enthalten, besonders bevorzugt ausgewählt aus Substanzen mit der INCI-Bezeichnung Biosaccharide Gum-1 (z.B. in dem Handelsprodukt Fucogel® von Solabia), Biosaccharide Gum-2 (z.B. in dem Handelsprodukt Rhamnosoft® von Solabia), Biosaccharide Gum-3 (z.B. in dem Handelsprodukt Fucogenol® von Solabia) und Biosaccharide Gum-4 (z.B. in dem Handelsprodukt Glycofilm® von Solabia), weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus® von Solabia. Weitere erfindungsgemäß bevorzugte Polysaccharide sind ausgewählt aus den Glycosaminoglycanen, besonders bevorzugt Hyaluronsäure, Dextran, Dextransulfat, Chondroitin-4-sulfat und Chondroitin-6-sulfat.

Weitere erfindungsgemäß bevorzugte Polysaccharide sind ausgewählt aus den Glucanen. Bei Glucanen oder Polyglucosanen handelt es sich um eine Klasse von Homopolysacchariden, deren Monomerbaustein ausschließlich Glucose ist. Das Glucose-Molekül kann α-glycosidisch oder β-glycosidisch verknüpft, unterschiedlich stark verzweigt oder linear angeordnet sein.

Beispiele für Glucane sind Cellulose, Amylose, Glycogen, Lichenin, Laminarin aus Algen, Pachyman aus Baumpilzen und Hefeglucane mit β-1,3-Bindung; Nigeran, ein aus Pilzen isoliertes Mycodextran (α-1,3-Glucan, α-1,4-Glucan) und Pustulan (β-1,6-Glucan). Dextran, ein primär α-1,6-gebundenes D-Glucan, ist das bedeutendste in dieser Polysaccharid-Gruppe. Weitere technisch interessante und erfindungsgemäß hervorragend geeignete Glucane sind Curdlan (β-1,3-D-Glucan), Pullulan (α-1,4-gebundenes und α-1,6-gebundenes D-Glucan) und Schizophyllan (β-1,3-Grundkette, β-1,6-Seitenkette).

Weitere erfindungsgemäß bevorzugte Polysaccharid-Wirkstoffe, deren Gegenwart eine unerwartete Wirkungssteigerung des mindestens einen Advanced Glycation Endproduct (AGEs) und/oder dessen Vorläufer bewirkt, sind ausgewählt aus beta-(1,3-1,4)-Glucanen. Besonders bevorzugt sind beta-(1,3-1,4)-Glucane, die zu etwa 70% beta-1,4-Glucosid-Verknüpfungen und zu etwa 30% beta-1,3-Glucosid-Verknüpfungen aufweisen. Derartige beta-(1,3-1,4)-Glucane sind bevorzugt erhältlich aus Extrakten aus Haferkörnern (Avena Sativa (Oat) Kernel Extract). Erfindungsgemäß besonders bevorzugte Extrakte aus Haferkörnern sind unter der Handelsbezeichnungen Drago Beta Glucan (02/060800) und SymGlucan von der Firma Symrise erhältlich.

Weitere erfindungsgemäß bevorzugte Polysaccharid-Wirkstoffe, deren Gegenwart eine unerwartete Wirkungssteigerung des mindestens einen Advanced Glycation Endproduct (AGEs) und/oder dessen Vorläufer bewirkt, sind ausgewählt aus chemisch modifizierten Cellulose-Derivaten, vorzugsweise Hydroxyalkylcellulosen wie Hydroxyethylcellulose, Hydroxypropylcellulose, Hydroxypropylmethylcellulose, Methylcellulose, Hydroxymethylcellulose, Carboxymethylcellulose, weiterhin insbesondere aus Stärken sowie Stärkeabbauprodukten wie Amylose und Amylopektin, chemisch und/oder thermisch modifizierte Stärken, z. B. Hydroxypropylstärkephosphat, Dihydroxypropyldistärkephosphat oder die Handelsprodukte Dry Flo®, weiterhin Chitosan und dessen Salzen und anderen Derivaten, weiterhin aus Polysacchariden, die Gums oder Gummen bilden, wie beispielsweise Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack und Agar-Agar.

Erfindungsgemäß besonders bevorzugte kosmetische oder dermatologische Zusammensetzungen sind dadurch gekennzeichnet, dass sie Maltosin und Biosaccharide Gum-1 enthalten. Weitere erfindungsgemäß besonders bevorzugte kosmetische oder dermatologische Zusammensetzungen sind dadurch gekennzeichnet, dass sie Maltosin und beta-(1,3-1,4)-Glucane enthalten.

Weitere erfindungsgemäß bevorzugte Wirkstoffe, deren Gegenwart eine unerwartete Wirkungssteigerung des mindestens einen Advanced Glycation Endproduct (AGEs) und/oder dessen Vorläufer bewirkt, sind ausgewählt aus hyperämisierenden Wirkstoffen.

Erfindungsgemäß bevorzugte kosmetische oder dermatologische Zusammensetzungen sind dadurch gekennzeichnet, dass sie mindestens einen hyperämisierenden Wirkstoff enthalten, der ausgewählt ist aus
- Nicotinsäureestern, insbesondere Nicotinsäurebenzylester (Benzylnicotinat), Nicotinsäurebutoxyethylester (Nicoboxil), Nicotinsäuremethylester, Nicotinsäureethylester, Nicotinsäurehexylester, Nicotinsäureisopropylester, Nicotinsäuremyristylester, Tetrahydrofurfuryl-Nicotinat (Thurfyl-Nicotinat) und Tetrahydrofurfuryl-Nicotinat-Hydrochlorid,
- Pyridin-3-carbaldehyd (β-Pyridincarbaldehyd, Nicotinaldehyd),
- Salicylsäureestern, insbesondere Phenylsalicylat,
- Vanillylethern, insbesondere Vanillylbutylether,
- Carbonsäurevanillylamiden, insbesondere Nonivamid (Nonansäurevanillylamid),
- Scharfstoffen, insbesondere Capsaicin, Cantharidin, Piperin, Gingerol, Zingeron, Myristicin, Safrol, Allicin, Sedamin, Sacculatal, Chalciporon, Isochalciporon, Velleral, Vellerol, und Isothiocyanaten (Senfölen), insbesondere Benzylsenföl,
- Extrakten aus Scharfstoff-Pflanzen, insbesondere aus Capsicum, insbesondere aus Capsicum annuum und Capsicum frutescens, aber auch aus Capsicum chinense, Capsicum baccatum und Capsicum pubescens, weiterhin aus Mauerpfeffer und aus der Zaunrübe,
- Terpentinöl,
- sowie Mischungen hiervon.

Bevorzugte Scharfstoffe bzw. Scharfstoff-Pflanzen sind: Capsaicin aus Paprika, Piperin aus Pfeffer, Gingerol und Zingeron aus Ingwer, Myristicin aus Muskat, Allicin aus Knoblauch, Sedamin aus Mauerpfeffer (Sedum-Alkaloid), Sacculatal aus Lebermoosen, Chalciporon aus dem Pfeffer-Röhrling (*Suillus piperatus*)*,* Velleral aus Milchlings-(*Lactarius*)-Arten, Isothiocyanate (Senföle) aus Senf, Meerrettich, Kresse und anderen Kreuzblütlern (Brassicaceae). Senföle ist die historisch bedingte Bezeichnung für organische Isothiocyanate, R-N=C=S, besonders für solche, die als stechend riechende, geschmacksgebende Bestandteile der etherischen Öle vorwiegend in Brassicaceae (Kreuzblütlern) vorkommen. Die Senföle liegen dort glycosidisch gebunden als Glucosinolate vor, aus denen sie - unter Umlagerung - durch Thioglucosidasen (Beispiel: Myrosinase) freigesetzt werden. Verbreitete Senföle sind Allylsenföl (Allylisothiocyanat), 3-Methylthiopropylsenföl, Butylsenföl, 3-Butenylsenföl, Erucin, Erysolin, Hirsutin, Isopropylsenföl, β-Phenylethylsenföl, Sulforaphan und Sulforaphen (Raphanin).

Entsprechende hyperämisierende Wirkstoffe sind im Handel erhältlich, teilweise in für kosmetische oder dermatologische Zusammensetzungen aufbereiteter Form. Erfindungsgemäß außerordentlich bevorzugte Wirkstoffe sind Ethylnicotinat, Vanillylbutylether, z.B. der Rohstoff Hotact VBE von Takasago, Capsaicin, erhältlich z. B. als Rohstoff Herbasol Extract Capsicum (INCI: Propylene Glycol, Aqua (Water), Capsicum Frutescens Fruit Extract, Sorbitol) und N-Vanillylnonamid (Pseudocapsaicin).

Bevorzugte erfindungsgemäße Zusammensetzungen sind dadurch gekennzeichnet, dass sie mindestens einen hyperämisierenden Wirkstoff in einer Aktivsubstanz-Gesamtmenge von 0,005 - 3,0 Gew.-%, bevorzugt 0,01 - 1,0, besonders bevorzugt 0,05 - 0,5 Gew.-% und außerordentlich bevorzugt 0,1 - 0,35 Gew.-%, jeweils bezogen auf das Gesamtgewicht der erfindungsgemäßen Zusammensetzung, enthalten.

Erfindungsgemäß besonders bevorzugte kosmetische oder dermatologische Zusammensetzungen sind dadurch gekennzeichnet, dass sie mindestens eine der folgenden Wirkstoffkombinationen enthalten:
Maltosin und 1,3,7-Trimethylxanthin, denn es wurde überraschend festgestellt, dass diese Kombination die Mikrozirkulation in den Zellen der Haut verbessert,
Maltosin und 1,3,7-Trimethylxanthin und Sojaproteinhydrolysat mit einer mittleren Molmasse (Mw) im Bereich von 1200 - 1800 Dalton, denn es wurde überraschend festgestellt, dass diese Kombination die Mikrozirkulation in den Zellen der Haut weiter steigern kann,
Maltosin und 1,3,7-Trimethylxanthin und N-Palmitoyl-Gly-His-Lys und N-Palmitoyl-Gly-Gln-Pro-Arg, denn es wurde überraschend festgestellt, dass diese Kombination die Mikrozirkulation in den Zellen der Haut weiter steigern kann,
Maltosin und 1,3,7-Trimethylxanthin und N-Palmitoyl-Lys-Thr-Thr-Lys-Ser, denn es wurde überraschend festgestellt, dass diese Kombination die Mikrozirkulation in den Zellen der Haut verbessert verbessert,
Maltosin und Carnitin,
Maltosin und 1,3,7-Trimethylxanthin und Carnitin, denn es wurde überraschend festgestellt, dass diese Kombination die Mikrozirkulation in den Zellen der Haut,
Maltosin und Carnitin und Sojaproteinhydrolysat mit einer mittleren Molmasse (Mw) im Bereich von 1200 - 1800 Dalton,
Maltosin und Carnitin und N-Palmitoyl-Gly-His-Lys und N-Palmitoyl-Gly-Gln-Pro-Arg,
Maltosin und Carnitin und N-Palmitoyl-Lys-Thr-Thr-Lys-Ser,
Maltosin und 1,3,7-Trimethylxanthin und Carnitin und Sojaproteinhydrolysat mit einer mittleren Molmasse (Mw) im Bereich von 1200 - 1800 Dalton und N-Palmitoyl-Gly-His-Lys und N-Palmitoyl-Gly-Gln-Pro-Arg,
Maltosin und 1,3,7-Trimethylxanthin und Carnitin und Sojaproteinhydrolysat mit einer mittleren Molmasse (Mw) im Bereich von 1200 - 1800 Dalton und N-Palmitoyl-Lys-Thr-Thr-Lys-Ser,
Maltosin und 3,7-Dimethylxanthin und Sojaproteinhydrolysat mit einer mittleren Molmasse (Mw) im Bereich von 1200 - 1800 Dalton,
Maltosin und 3,7-Dimethylxanthin und N-Palmitoyl-Gly-His-Lys und N-Palmitoyl-Gly-Gln-Pro-Arg,
Maltosin und 3,7-Dimethylxanthin und N-Palmitoyl-Lys-Thr-Thr-Lys-Ser,
Maltosin und 3,7-Dimethylxanthin und Carnitin,
Maltosin und Carnitin und Sojaproteinhydrolysat mit einer mittleren Molmasse (Mw) im Bereich von 1200 - 1800 Dalton,
Maltosin und Carnitin und N-Palmitoyl-Gly-His-Lys und N-Palmitoyl-Gly-Gln-Pro-Arg,
Maltosin und Carnitin und N-Palmitoyl-Lys-Thr-Thr-Lys-Ser,
Maltosin und 3,7-Dimethylxanthin und Carnitin und Sojaproteinhydrolysat mit einer mittleren Molmasse (Mw) im Bereich von 1200 - 1800 Dalton und N-Palmitoyl-Gly-His-Lys und N-Palmitoyl-Gly-Gln-Pro-Arg,
Maltosin und 3,7-Dimethylxanthin und Carnitin und Sojaproteinhydrolysat mit einer mittleren Molmasse (Mw) im Bereich von 1200 - 1800 Dalton und N-Palmitoyl-Lys-Thr-Thr-Lys-Ser,
Maltosin und 1,3-Dimethylxanthin und Sojaproteinhydrolysat mit einer mittleren Molmasse (Mw) im Bereich von 1200 - 1800 Dalton,
Maltosin und 1,3-Dimethylxanthin und N-Palmitoyl-Gly-His-Lys und N-Palmitoyl-Gly-Gln-Pro-Arg,
Maltosin und 1,3-Dimethylxanthin und N-Palmitoyl-Lys-Thr-Thr-Lys-Ser,
Maltosin und 1,3-Dimethylxanthin und Carnitin,
Maltosin und Carnitin und Sojaproteinhydrolysat mit einer mittleren Molmasse (Mw) im Bereich von 1200 - 1800 Dalton,
Maltosin und Carnitin und N-Palmitoyl-Gly-His-Lys und N-Palmitoyl-Gly-Gln-Pro-Arg,
Maltosin und Carnitin und N-Palmitoyl-Lys-Thr-Thr-Lys-Ser,
Maltosin und 1,3-Dimethylxanthin und Carnitin und Sojaproteinhydrolysat mit einer mittleren Molmasse (Mw) im Bereich von 1200 - 1800 Dalton und N-Palmitoyl-Gly-His-Lys und N-Palmitoyl-Gly-Gln-Pro-Arg,
Maltosin und 1,3-Dimethylxanthin und Carnitin und Sojaproteinhydrolysat mit einer mittleren Molmasse (Mw) im Bereich von 1200 - 1800 Dalton und N-Palmitoyl-Lys-Thr-Thr-Lys-Ser,
Maltosin und Capsicum Frutescens Fruit Extrakt, denn es wurde überraschend festgestellt, dass diese Kombination die Mikrozirkulation in den Zellen der Haut verbessert,
Maltosin und Ethylnicotinat, denn es wurde überraschend festgestellt, dass diese Kombination die Mikrozirkulation in den Zellen der Haut verbessert,
Maltosin und Vanillylbutylether, denn es wurde überraschend festgestellt, dass diese Kombination die Mikrozirkulation in den Zellen der Haut verbessert,
Maltosin und N-Vanillylnonamid, denn es wurde überraschend festgestellt, dass diese Kombination die Mikrozirkulation in den Zellen der Haut verbessert.

Es kann erfindungsgemäß besonders bevorzugt sein, dass die als Wirkstoff eingesetzten Aminosäuren und Peptide zur Verbesserung der Penetration in die Haut mit mindestens einer, bevorzugt linearen, C₂-C₂₂-Fettsäure N-acyliert und/oder verestert sind. Zur N-Acylierung und/oder Veresterung besonders bevorzugt sind C₈-C₁₈-Fettsäuren, ganz besonders bevorzugt sind Myristinsäure (C₁₄) und Palmitinsäure (C₁₆). Besonders bevorzugt ist weiterhin, dass alle verwendeten Aminosäuren und Peptide derartig N-acyliert und/oder verestert sind. Ebenfalls bevorzugt ist die Substitution der Aminosäuren und Peptide mit einer Benzyloxycarbonylgruppe an der terminalen Aminogruppe.

In einer weiteren bevorzugten Ausführungsform der Erfindung liegen die Monomeren und Oligomeren von Aminosäuren, N-C₂-C₂₄-Acylaminosäuren, den Estern und/oder den physiologisch verträglichen Metallsalzen dieser Substanzen in geträgerter Form vor, insbesondere aufgetragen auf feinteiligen, pulverförmigen Substraten wie Kieselgel, insbesondere Aerosil-Typen, weiterhin Talkum, Microsponges, modifizierten Stärken und Stärkederivaten, kristalliner Cellulose, Cellulosepulvern, Lactoglobulinderivaten, Polymerpartikeln aus Nylon, Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon und Siliconen. Ein besonders bevorzugter Rohstoff dieser Art sind die Vegetal Filling Spheres von Coletica.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen weiteren, die Collagensynthese stimulierenden Wirkstoff enthalten, der bevorzugt ausgewählt ist aus Retinoiden, insbesondere Retinol und C₂-C₂₂-Fettsäureestern des Retinols, Oleanolsäure, Oleanol, Kreatin, sowie Mischungen der vorgenannten Substanzen.

Weitere erfindungsgemäß bevorzugte Wirkstoffe, die die Collagensynthese stimulieren, sind ausgewählt aus Retinoiden. Zu den Retinoiden werden im Sinne der vorliegenden Erfindung Retinol (Vitamin A₁), die C₂-C₂₂-Fettsäureester des Retinols (= Retinylester, insbesondere Retinylpalmitat und Retinylacetat), 3,4-Didehydroretinol (Vitamin A₂), Retinal und seine Isomere, *all*-*trans*-Retinsäure, 9-*cis-*Retinsäure und 13-*cis*-Retinsäure (Tretinoin), die Ester der Retinsäure sowie weitere verwandte Substanzen (synthetische Retinoide) gezählt, die für ihre vielfältige biologische Wirkung insbesondere auf Wachstum und Differenzierung bekannt sind, sowie weiterhin Substanzen, die eine spezifische biologische Wirkung durch die Bindung an die Retinoid-Rezeptoren RAR (retinoid acid receptor) und RXR (retinoid X receptor) aufweisen und deren Aktivierung verursachen. Synthetische Retinoide werden in drei Gruppen eingeteilt: nichtaromatisch (z. B. *Isotretinoin*), monoaromatisch (z.B. Acitretin) und polyaromatisch (sogenannte Arotinoide, z. B. Tazarotene, die eine spezifische Wirkung auf einzelne Retinoid-Rezeptoren haben). Erfindungsgemäß besonders bevorzugte Retinoide sind Retinol und die C₂-C₂₂-Fettsäureester des Retinols, insbesondere Retinylpalmitat und Retinylacetat.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen Wirkstoff, der die Collagensynthese stimuliert, in einer Gesamtmenge von 0,000001 - 5 Gew.-%, bevorzugt 0,00001 - 2 Gew.-%, besonders bevorzugt 0,0001 - 1 Gew.-% und außerordentlich bevorzugt 0,005 - 0,5 Gew.-%, enthalten, jeweils bezogen auf den Gehalt an Aktivsubstanz in der gesamten Zusammensetzung.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen weiteren Wirkstoff enthalten, der ausgewählt ist aus Vitaminen, Provitaminen und Vitaminvorstufen der Gruppen B und E und den Estern der vorgenannten Substanzen, α-Hydroxycarbonsäuren, α-Ketocarbonsäuren, β-Hydroxycarbonsäuren und deren Ester-, Lacton- und/oder Salzform, Flavonoiden und Flavonoid-reichen Pflanzenextrakten, Ubichinon und Ubichinol sowie deren Derivaten, Silymarin, Ectoin, anorganischen und organischen UV-Filtersubstanzen, selbstbräunenden, hautberuhigenden Wirkstoffen sowie Mischungen dieser Wirkstoffe.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen Wirkstoff enthalten, der ausgewählt ist aus mindestens einem Vitamin, Provitamin oder einer als Vitaminvorstufe bezeichnete Verbindung aus den Vitamingruppen B und E und den Estern der vorgenannten Substanzen.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte Zusammensetzung, eingesetzt.
- Vitamin B₂, Trivialname Riboflavin, chemische Bezeichnung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf die gesamte Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (VIT-I) eingesetzt werden.

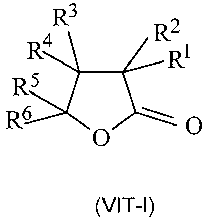

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (VIT-I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.

Die genannten Verbindungen des Vitamin B₅-Typs sowie die 2-Furanonderivate sind in den erfindungsgemäßen Mitteln in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methyl-pyridin-3-ols versteht. Vitamin B₆ ist in den erfindungsgemäßen Mitteln bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3aS,4S, 6a*R*)-2-Oxohexahydrothienol[3,4-*d*]-imidazol-4-valeriansäure. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.

Erfindungsgemäß besonders bevorzugte kosmetische oder dermatologische Zusammensetzungen sind dadurch gekennzeichnet, dass sie mindestens eine der folgenden Wirkstoffkombinationen enthalten:
Maltosin und Folsäure, denn es wurde überraschend festgestellt, dass diese Kombination die haarwuchsinhibierende Wirkung des Maltosins verbessert,
Maltosin und 1,3,7-Trimethylxanthin und Folsäure, denn es wurde überraschend festgestellt, dass diese Kombination die Mikrozirkulation in den Zellen der Haut weiter steigern kann,
Maltosin und Folsäure und N-Palmitoyl-Gly-His-Lys und N-Palmitoyl-Gly-Gln-Pro-Arg, denn es wurde überraschend festgestellt, dass diese Kombination die Mikrozirkulation in den Zellen der Haut weiter steigern kann,
Maltosin und Folsäure und N-Palmitoyl-Lys-Thr-Thr-Lys-Ser, denn es wurde überraschend festgestellt, dass diese Kombination die Mikrozirkulation in den Zellen der Haut verbessert, Maltosin und Carnitin und Folsäure.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen weiteren Wirkstoff, ausgewählt aus mindestens einer α-Hydroxycarbonsäure, α-Ketocarbonsäure oder β-Hydroxycarbonsäure oder deren Ester-, Lacton- und/ oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Weitere Derivate der vorgenannten Säuren sind deren physiologisch verträglichen Salze, bevorzugt die Zink-, Kupfer- und Mangansalze, die Salze der Alkali- und der Erdalkalimetalle und die Ammonium-, Alkylammonium-, Alkanolammonium- und Glucammoniumsalze, besonders bevorzugt die Natrium-, Kalium-, Magnesium- und Calcium-Salze.

Die α-Hydroxycarbonsäuren, α-Ketocarbonsäuren oder β-Hydroxycarbonsäuren oder ihre Derivate sind bevorzugt in einer Gesamtmenge von 0,1 - 10 Gew.-%, besonders bevorzugt 0,5 - 5 Gew.-% und außerordentlich bevorzugt 1 - 2 Gew.-%, jeweils bezogen auf die gesamte Zusammensetzung, enthalten.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen weiteren Wirkstoff, ausgewählt aus mindestens einem Flavonoid und/oder mindestens einem Flavonoid-reichen Pflanzenextrakt.

Die erfindungsgemäß bevorzugten Flavonoide umfassen die Glycoside der Flavone, der 3-Hydroxyflavone (Flavonole) und der Aurone. Die Flavanone und die Isoflavone sind erfindungsgemäß ausdrücklich von den Flavonoiden ausgenommen. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)flavon-3-(6-O-(6-deoxy-α-L-mannopyranosyl)-β-D-glucopyranosid)), Eriodictin und Apigenin-7-glucosid (4',5,7-Trihydroxyflavon-7-glucosid). Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.

Erfindungsgemäß werden die Flavonoide in Mengen von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, eingesetzt.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen weiteren Wirkstoff, ausgewählt aus mindestens einem Ubichinon oder einem Ubichinol oder deren Derivaten. Ubichinole sind die reduzierte Form der Ubichinone. Die erfindungsgemäß bevorzugten Ubichinone weisen die Formel (UBI-I) auf: 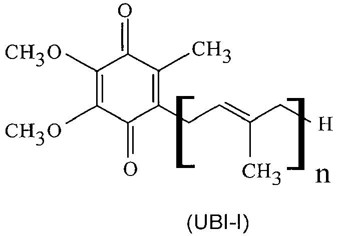 mit n = 6, 7, 8, 9 oder 10. Besonders bevorzugt ist das Ubichinon der Formel (UBI-I) mit n = 10, auch bekannt als Coenzym Q10. Erfindungsgemäß werden die Ubichinone, Ubichinole oder deren Derivate in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt. Erfindungsgemäß besonders bevorzugte kosmetische oder dermatologische Zusammensetzungen sind dadurch gekennzeichnet, dass sie mindestens eine der folgenden Wirkstoffkombinationen enthalten:
Maltosin und Coenzym Q 10, denn es wurde überraschend festgestellt, dass diese Kombination die Mikrozirkulation in den Zellen der Haut verbessert,
Maltosin und 1,3,7-Trimethylxanthin und Coenzym Q 10, denn es wurde überraschend festgestellt, dass diese Kombination die haarwuchsinhibierende Wirkung in überraschend hohem Maße verbessern kann,
Maltosin und Carnitin und Coenzym Q 10, denn es wurde überraschend festgestellt, dass diese Kombination die haarwuchsinhibierende Wirkung in unerwartet hohem Maße verbessern kann.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen weiteren Wirkstoff, ausgewählt aus Silymarin, enthalten. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.

Erfindungsgemäß wird Silymarin in Mengen von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, eingesetzt.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen weiteren Wirkstoff, ausgewählt aus Ectoin, enthalten. Ectoin ist der Trivialname für 2-Methyl-1,4,5,6-tetrahydropyrimidin-4-carboxylat. Erfindungsgemäß ist Ectoin bevorzugt in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

Erfindungsgemäß besonders bevorzugte kosmetische oder dermatologische Zusammensetzungen sind dadurch gekennzeichnet, dass sie mindestens eine der folgenden Wirkstoffkombinationen enthalten:
Maltosin und Ectoin, denn es wurde überraschend festgestellt, dass diese Kombination die haarwuchsinhibierende Wirkung verbessert,
Maltosin und 1,3,7-Trimethylxanthin und Ectoin, denn es wurde überraschend festgestellt, dass diese Kombination die haarwuchsinhibierende Wirkung in überraschend hohem Maße verbessern kann.

Weitere erfindungsgemäß bevorzugte kosmetische oder dermatologische Zusammensetzungen sind dadurch gekennzeichnet, dass sie mindestens ein Advanced Glycation Endproduct (AGEs) und/oder dessen Vorläufer und mindestens einen weiteren Wirkstoff, ausgewählt aus mindestens einer anorganischen und/oder mindestens einer organischen UV-Filtersubstanz enthalten.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt. Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein.

Erfindungsgemäß sind die organischen UV-Filtersubstanzen bevorzugt in Mengen von 0,1 - 30 Gew.-%, besonders bevorzugt 0,5 - 20 Gew.-%, außerordentlich bevorzugt 1,0 - 15 Gew.-% und weiter bevorzugt 3,0 - 10 Gew.-%, jeweils bezogen auf die gesamte Zusammensetzung, enthalten. Erfindungsgemäß sind die anorganischen UV-Filtersubstanzen bevorzugt in Mengen von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen weiteren Wirkstoff, ausgewählt aus mindestens einem selbstbräunenden Wirkstoff enthalten. Erfindungsgemäß bevorzugte selbstbräunende Wirkstoffe sind ausgewählt aus Dihydroxyaceton, Erythrulose und 5,6-Dihydroxyindolin. Erfindungsgemäß sind die selbstbräunenden Wirkstoffe in einer Gesamtmenge von 0,01 - 15 Gew.-%, bevorzugt 0,1 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen weiteren Wirkstoff, ausgewählt aus hautberuhigenden Wirkstoffen, enthalten. Erfindungsgemäß bevorzugte hautberuhigende Wirkstoffe sind ausgewählt aus Allantoin, α-Bisabolol, α-Liponsäure, Glycyrrethinsäure, die besonders bevorzugt in Liposomen verkapselt vorliegt und in dieser Form z. B. unter dem Handelsnamen Calmsphere von Soliance erhältlich ist, den physiologisch verträglichen Salzen von Sterolsulfaten, wie sie z. B. unter dem Handelsnamen Phytocohesine (INCI: Sodium Beta-Sitosterylsulfate) von der Firma Vincience erhältlich sind, Aminodicarbonsäuren mit einer C-Kettenlänge von 3 - 6 Kohlenstoffatomen sowie deren physiologisch verträglichen Salzen, bevorzugt ausgewählt aus Aminomalonsäure, Aminobernsteinsäure (= Asparaginsäure), Aminoglutarsäure und Aminoadipinsäure sowie deren physiologisch verträglichen Salzen wie Kaliumaspartat und Magnesiumaspartat, Extrakten aus den Samen von Cucurbita pepo (Zucchini), Extrakten aus Mentha piperita, insbesondere aus den Blättern von Mentha piperita, N-acylierten Alkanolaminen, insbesondere N-Palmitoylethanolamin, weiterhin ausgewählt aus dem Dipeptid Tyr-Arg, dessen Estern und dessen N-Acyl-Derivaten, insbesondere N-Acetyl-Tyr-Arg-hexyldecylester (z. B. Calmosensine von Sederma, sowie beliebigen Mischungen dieser Substanzen.

Erfindungsgemäß besonders bevorzugte Extrakte aus den Samen von Cucurbita pepo (Zucchini) sind beispielsweise in den Handelsprodukten Curbilene (ex Indena SpA, INCI: Cucurbita pepo (pumpkin) seed extract), Ocaline (ex Soliance, INCI: Sea Water (and) Water (and) Cucurbita pepo (pumpkin) seed extract), ARP 100 (ex Greentech, INCI: Water, Alcohol, Serenoa Serrulata Fruit Extract, Epilobium Angustifolium Extract, Cucurbita Pepo (Pumpkin) Seed Extract), ARP 100 Huileux (ex Greentech, INCI: Caprylic/Capric Triglyceride, Serenoa Serrulata Fruit Extract, Epilobium Angustifolium Flower/Leaf/Stem Extract, Cucurbita Pepo (Pumpkin) Seed Extract), Cucurbitine (ex Christian Dior Parfums, INCI: Water (and) Cucurbita pepo (pumpkin) seed extract), Herbasol Extract Pumpkin (ex Cosmetochem, INCI: Water (and) Propylene glycol (and) Cucurbita pepo (pumpkin) seed extract) und Pumpkin Extract (ex Draco Natural Products, INCI: Water (and) Cucurbita pepo (pumpkin) seed extract) enthalten.

Erfindungsgemäß besonders bevorzugte Extrakte aus Mentha piperita, insbesondere aus den Blättern von Mentha piperita, sind beispielsweise in den Handelsprodukten Calmiskin OP (ex Silab, INCI: Water (and) Mentha piperita (Peppermint) Leaf Extract) und Caomint (ex Solabia, INCI: Propylene Glycol, Water, Mentha piperita (Peppermint) Leaf Extract, Theobroma Cacao (Cocoa) Extract) enthalten.

Weitere erfindungsgemäß bevorzugte kosmetische oder dermatologische Zusammensetzungen enthalten Glycyrrethinsäure zusätzlich zu mindestens einem Advanced Glycation Endproduct (AGEs) und/oder dessen Vorläufer.

Der mindestens eine hautberuhigende Wirkstoff ist bevorzugt in einer Gesamtmenge von 0,001 bis 5 Gew.-%, besonders bevorzugt 0,01 bis 2 Gew.-% und außerordentlich bevorzugt 0,1 bis 1 Gew.- %, jeweils bezogen auf die gesamte Zusammensetzung, enthalten.

Ein weiterer Gegenstand der vorliegenden Erfindung ist die nicht-therapeutische, kosmetische Verwendung einer kosmetischen oder dermatologischen Zusammensetzung gemäß einem der Ansprüche 1 - 13 zur haarwuchsinhibierenden Behandlung der Haut.

Weitere erfindungsgemäß bevorzugte kosmetische oder dermatologische Zusammensetzungen sind dadurch gekennzeichnet, dass sie zusätzlich zu mindestens einem Advanced Glycation Endproduct (AGEs) und/oder dessen Vorläufer mindestens einen weiteren Wirkstoff, ausgewählt aus mindestens einem konditionierenden Wirkstoff, enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen. Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆₋₃₀-Fettsäuren mit C₂₋₃₀- Fettalkoholen-Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol® CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder HydroxyGruppen enthalten, bevorzugt Substanzen mit den INCI-Bezeichnungen Dimethiconol, Amodimethicone oder Trimethylsilylamodimethicone. Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf das gesamte Hautbehandlungsmittel.

Die erfindungsgemäßen kosmetischen oder dermatologischen Zusammensetzungen liegen in Form einer flüssigen, fließfähigen oder festen ÖI-in-Wasser-Emulsion, Wasser-in-ÖI-Emulsion, Mehrfach-Emulsion, insbesondere einer ÖI-in-Wasser-in-ÖI- oder Wasser-in-ÖI-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, eines Schaumes, eines Puders oder einer Mischung mit mindestens einem als medizinischen Klebstoff geeigneten Polymer vor.

In der bevorzugten Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov® 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls® PGPH) und Polyglyceryl-3-diisostearat (Handelsprodukt Lameform® TGI) sowie lineare und verzweigte C₈-C₃₀-Fettsäuren und deren Na-, K-, Ammonium-, Ca-, Mg- und Zn - Salze. Die erfindungsgemäßen Zusammensetzungen enthalten die Emulgatoren bevorzugt in Mengen von 0,1 bis 25 Gew.-%, besonders bevorzugt 0,5 - 15 Gew.-%, bezogen auf die gesamte Zusammensetzung.

In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel R¹ - O - R², in der R¹ eine primäre lineare Alkyl-, Alkenyl- oder Acylgruppe mit 20 - 30 C-Atomen, bevorzugt ein Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt. Eine besonders bevorzugte Verbindung R¹ - O - R² ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythritmonoerucat. Die Anwesenheit von Emulgatoren der allgemeinen Formel R¹ - O - R² kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher dadurch gekennzeichnet, dass sie in Form einer lamellare Strukturen aufweisenden Öl-in-Wasser-Emulsion vorliegen.

In einer weiteren besonders bevorzugten Ausführungsform liegen die erfindungsgemäßen Zusammensetzungen in Form einer Wasser-in-Öl-Emulsion vor. Derartige Wasser-in-Öl-Emulsionen können so formuliert werden, dass die eine höhere Viskosität aufweisen, so dass sie sorgfältig in die Haut einzumassieren sind. Dies unterstützt die haarwuchsinhibierende Wirkung der erfindungsgemäßen Zusammensetzungen. Genau so gut lassen sich aber auch niedrig-viskose Wasser-in-Öl-Emulsionen formulieren, insbesondere auf Basis des polymeren Wasser-in-Öl-Emulgators PEG-30 Dipolyhydroxystearat, erhältlich z. B. unter dem Handelsnamen Arlacel P 135 von Uniqema. Insbesondere mit Hilfe dieses Emulgators lassen sich sprühbare Wasser-in-Öl-Emulsionen formulieren.

Weitere geeignete Zusatzstoffe sind Verdickungsmittel, z. B. natürliche und synthetische Tone und Schichtsilikate wie Bentonit, Hectorit, Montmorillonit oder Laponite®, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel®305, Simulgel®600, Simulgel® NS und Simulgel® EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol®. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen® und die Carbopol®-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol® sowie Vinylpyrrolidon/Vinylester-Copolymere und Polyvinylpyrrolidone, die z. B. unter dem Warenzeichen Luviskol® (BASF) vertrieben werden.

Weitere bevorzugte Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum®, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

Da die erfindungsgemäßen Zusammensetzungen insbesondere für die (großflächige) Anwendung auf der Körperhaut bestimmt sind, ist es besonders vorteilhaft und praktisch, wenn sie als sprühbare Zusammensetzung vorliegen. Ein weiterer Gegenstand der vorliegenden Erfindung ist daher eine Zusammensetzung gemäß einem der Ansprüche 1 - 13, die in einem Sprühspender oder Pumpspender verpackt ist.

Ein weiterer Gegenstand der vorliegenden Erfindung ist die nicht-therapeutische, kosmetische Verwendung einer erfindungsgemäßen kosmetischen oder dermatologischen Zusammensetzung zur Regulierung des Haarwuchses, insbesondere zur Verringerung des Haarwuchses Bezüglich bevorzugter Ausführungsformen der erfindungsgemäßen Verwendungen gilt mutatis mutandis das zu den erfindungsgemäßen Zusammensetzungen Gesagte.

### Ausführungsbeispiele

Die nachfolgenden Formulierungsbeispiele sollen den Gegenstand der Erfindung erläutern, ohne ihn hierauf zu beschränken.

Alle Mengenangaben sind in Gew.-%, bezogen auf die gesamte Zusammensetzung.

### 1. Öl-in-Wasser-Emulsionen

### 1. Hautcremes

Die Zusammensetzungen 1.1 - 1.4 sind bevorzugte Ausführungsformen von pflegenden Tagescremes mit haarwuchsinhibierendem Effekt.

| | 1.1 | 1.2 | 1.3 | 1.4 |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearvl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Dimethicone (350 cSt) | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| Algenextrakt | 1,00 | - | - | - |
| Caomint | 1,00 | 0,50 | 2,00 | - |
| Calmosensine | 1,00 | 2,00 | - | 2,00 |
| Symdiol 68 | 0,30 | 0,50 | - | - |
| DSH-CN | 5,00 | 5,00 | 5,00 | 5,00 |
| Hydrovance | 4,30 | 2,50 | 8,60 | 10,00 |
| Kombuchka | 3,00 | - | - | 3,00 |
| Glistin | - | 2,00 | - | - |
| Natrulon RC 50 DG | - | - | 1,00 | - |
| Phytokine | 2,00 | 1,50 | 2,00 | 2,00 |
| Ridulisse C | 0,50 | 0,50 | 0,50 | 0,50 |
| Liftiline | 2,00 | 3,00 | 2,00 | 3,00 |
| Matrixyl 3000 | - | 2,00 | - | - |
| Olea europ. Fol extr. S. sicc. | - | - | - | 0,10 |
| Maltosin | 0,1 | 0,2 | 0,5 | 1,0 |
| Perfume | 0,10 | 0,10 | 0,10 | 0,10 |
| Aqua | ad 100 | ad 100 | ad 100 | ad 100 |

### 2. Hautcremes:

Die Zusammensetzungen 2.1 - 2.3 sind bevorzugte Ausführungsformen von Tagescremes für besonders anspruchsvolle Haut mit einem haarwuchsinhibierenden Effekt und mit einem Gehalt an Antiageing-Wirkstoffen.

| | 2.1 | 2.2 | 2.3 |
|---|---|---|---|
| Cetearyl Isononanoate | 4,00 | 4,00 | 4,00 |
| Mineral oil | 6,00 | 6,00 | 6,00 |
| Cutina CBS | 2,00 | 2,00 | 2,00 |
| Palmitic Acid / Stearic Acid | 1,50 | 1,50 | 1,50 |
| Eumulgin B3 | 1,00 | 1,00 | 1,00 |
| Dimethicone (350 cSt) | 1,00 | 1,00 | 1,00 |
| Tocopheryl Acetate | 0,50 | 0,50 | 0,50 |
| Propylparaben | 0,30 | 0,30 | 0,30 |
| Pemulen TR 1 | 0,27 | 0,27 | 0,27 |
| Glycerin | 5,00 | 3,00 | 3,00 |
| Milchsäure 80 % | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Phytokine | 2,00 | 1,50 | 2,00 |
| Ridulisse C | 0,50 | 0,50 | 0,50 |
| Liftiline | 2,00 | 3,00 | 2,00 |
| Sepigel 305 | 0,50 | 0,50 | 0,50 |
| Silk Protein | 0,25 | 0,25 | 0,25 |
| Taurin | 1,00 | 0,50 | 2,00 |
| Calmosensine | 1,00 | 2,00 | - |
| Keltrol SF | 0,20 | 0,20 | 0,20 |
| N, N-Bis-(2-hydroxyethyl)harnstoff | 4,30 | 2,50 | 8,60 |
| Perfume | 0,30 | 0,30 | 0,30 |
| Natrium Ascorbylphosphat | 1,00 | - | 1,00 |
| Keratec Pep | - | 2,00 | - |
| Kreatin | - | - | 1,00 |
| Retinol | 0,10 | - | - |
| Deliner | - | 2,00 | - |
| Maltosin | 1,0 | 0,25 | 0,5 |
| Bodyfit | - | 3,00 | 2,50 |
| Wasser | ad 100 | ad 100 | ad 100 |

### 3. Tagescremes:

Die Zusammensetzungen 3.1 - 3.5 sind bevorzugte Ausführungsformen von Tagescremes für besonders anspruchsvolle Haut mit einem haarwuchsinhibierenden Effekt.

| | 3.1 | 3.2 | 3.3 | 3.4 | 3.5 |
|---|---|---|---|---|---|
| Lipoid S 75-3 | 0,50 | 0,50 | 0,50 | 0,50 | 0,50 |
| Isopropylstearate | 4,00 | 4,00 | 4,00 | 4,00 | 4,00 |
| Dibutyl Adipate | 2,00 | 2,00 | 2,00 | 2,00 | 2,00 |
| Tocopheryl Acetate | 0,50 | 0,50 | 0,50 | 0,50 | 0,50 |
| Cutina MD | 1,00 | 1,00 | 1,00 | 1,00 | 1,00 |
| Behenyl Alcohol | 2,00 | 2,00 | 2,00 | 2,00 | 2,00 |
| Dimethicone (350 cSt) | 0,50 | 0,50 | 0,50 | 0,50 | 0,50 |
| Propylparaben | 0,20 | 0,20 | 0,20 | 0,20 | 0,20 |
| Dow Corning 9040 | 1,00 | 1,00 | 1,00 | 1,00 | 1,00 |
| Glycerol | 3,00 | 4,50 | 3,00 | 3,00 | 3,00 |
| Hexandiol | 6,00 | 3,00 | - | - | 4,00 |
| Methylparaben | 0,20 | 0,20 | 0,20 | 0,20 | 0,20 |
| Tego Carbomer | 0,30 | 0,30 | 0,30 | 0,30 | 0,30 |
| Algae Extract | 1,00 | - | - | - | - |
| Photosomes | 0,20 | 0,20 | 0,20 | 0,20 | 0,20 |
| Dimethylmethoxy Chromanol | 0,01 | 0,01 | 0,01 | 0,01 | 0,01 |
| Propylenglycol | 5,00 | 5,00 | 5,00 | 5,00 | 5,00 |
| Phytokine | 2,00 | 1,50 | 2,00 | 2,00 | 1,50 |
| Ridulisse C | 0,50 | 0,50 | 0,50 | 0,50 | 0,50 |
| Liftiline | 2,00 | 3,00 | 2,00 | 2,00 | 3,00 |
| Caomint | 1,00 | - | 2,00 | 2,00 | 2,00 |
| Calmosensine | 1,00 | 2,00 | - | 1,00 | 1,00 |
| Kombuchka | 3,00 | 3,00 | - | - | 2,00 |
| Natrium Ascorbylphosphat | - | - | 1,00 | 1,00 | 1,00 |
| Maltosin | 0,1 | 0,2 | 0,5 | 1,0 | 2,0 |
| Natrulon RC50DG | - | - | - | - | 1,00 |
| Deliner | - | - | 2,00 | 1,00 | - |
| Matrixyl 3000 | 1,00 | 1,00 | - | - | 1,00 |
| Simulgel NS | 1,50 | 1,50 | 1,50 | 1,50 | 1,50 |
| Sodium Benzoate | - | 0,20 | 0,50 | - | - |
| Phenoxyethanol | 0,50 | - | - | - | - |
| Symdiol 68 | 0,30 | 0,50 | 0,30 | - | - |
| TiO₂ | 0,50 | 0,50 | - | - | - |
| Hydrovance | 2,50 | 4,30 | 8,60 | 8,60 | 8,60 |
| Silymarin Phytosome | 0,30 | - | - | - | 0,50 |
| Ectoin | 0,50 | - | 0,50 | 0,50 | 0,50 |
| Vitamin B6 | - | - | 0,01 | 0,01 | 0,01 |
| Perfume | 0,35 | 0,35 | 0,35 | 0,35 | 0,35 |
| Aqua | ad 100 | ad 100 | ad 100 | ad 100 | ad 100 |

Die Zusammensetzungen 3.6 - 3.11 sind bevorzugte Ausführungsformen von pflegenden Cremes mit gelähnlicher Konsistenz mit einem haarwuchsinhibierenden Effekt.

| | 3.6 | 3.7 | 3.8 | 3.9 | 3.10 | 3.11 |
|---|---|---|---|---|---|---|
| 2-Ethylhexylpalmitat | 2,00 | 2,00 | 2,00 | 2,00 | 2,00 | 2,00 |
| Dow Corning 1403 Fluid | 5,00 | 5,00 | 5,00 | 5,00 | 5,00 | 5,00 |
| Synperonic PE L 64 | 2,00 | 2,00 | 2,00 | 2,00 | 2,00 | 2,00 |
| Dow Corninq 9040 | 5,00 | 5,00 | 5,00 | 5,00 | 5,00 | 5,00 |
| Dipropylenglykol | 5,00 | 5,00 | 5,00 | 5,00 | 5,00 | 5,00 |
| Tego Carbomer 140 | 0,70 | 0,70 | 0,70 | 0,70 | 0,70 | 0,70 |
| Parfum | 0,35 | 0,30 | 0,35 | 0,35 | 0,30 | 0,30 |
| Phenoxyethanol, rein | 0,40 | 0,40 | 0,40 | 0,40 | 0,40 | 0,40 |
| Natrulon RC-50DG | - | 0,75 | 0,75 | 0,75 | 0,75 | 0,75 |
| Menthyl Lactate | 0,50 | 0,50 | 0,50 | 0,50 | 0,50 | 0,50 |
| Ethanol 96 % DEP vergällt | 5,00 | 5,00 | 5,00 | 5,00 | 5,00 | 5,00 |
| Coffein wasserfrei | 0,50 | - | 0,50 | 0,50 | 0,50 | 0,50 |
| Ridulisse C | - | - | - | 0,50 | - | - |
| Maltosin | 0,5 | 1,0 | 1,5 | 0,1 | 0,2 | 0,5 |
| Simulgel NS | 0,75 | 0,75 | 0,75 | 0,75 | 0,75 | 0,75 |
| Matrixyl | - | - | - | - | 2,00 | - |
| Matrixyl 3000 | - | - | - | - | - | 2,00 |
| Natriumhydroxid Perlen | 0,081 | 0,081 | 0,081 | 0,081 | 0,081 | 0,081 |
| Wasser, vollentsalzt | ad 100,00 | ad 100,00 | ad 100,00 | ad 100,00 | ad 100,00 | ad 100,00 |

### 4. Beispielserie:

4.1 - 4.5 sind bevorzugte Ausführungsformen von Tagescremes mit höherem Lichtschutzfilter (SPF ca. 10 - 15) mit haarwuchsinhibierendem Effekt für besonders anspruchsvolle Haut.

| | 4.1 | 4.2 | 4.3 | 4.4 | 4.5 |
|---|---|---|---|---|---|
| Montanov 68 | 5,00 | 5,00 | 5,00 | 5,00 | 5,00 |
| Myritol 318 | 5,00 | 5,00 | 5,00 | 5,00 | 5,00 |
| Cetiol SB 45 | 0,50 | 0,50 | 0,50 | 0,50 | 0,50 |
| Novata AB | 2,00 | 2,00 | 2,00 | 2,00 | 2,00 |
| Stenol 1618 | 1,00 | 1,00 | 1,00 | 1,00 | 1,00 |
| Dimethicone (350 cSt) | 0,50 | 0,50 | 0,50 | 0,50 | 0,50 |
| Tocopherylacetat | 0,50 | 0,50 | 0,50 | 0,50 | 0,50 |
| Controx KS | 0,25 | 0,25 | 0,25 | 0,25 | 0,25 |
| Polvsilicone-15 | - | 4,00 | 4,00 | - | - |
| Phenylbenzimidazole sulfonic acid | - | 2,00 | 2,00 | 2,00 | - |
| Neo Heliopan AP | - | - | - | 1,00 | - |
| Octocrvlene | - | - | - | 5,00 | - |
| Uvinul MBC 95 | 2,00 | - | - | - | - |
| Butyl Methoxydibenzoylmethane | 1,00 | 1,80 | 1,80 | - | - |
| Propylparaben | 0,20 | 0,20 | 0,20 | 0,20 | 0,20 |
| Tego Carbomer 140 | 0,50 | 0,50 | 0,50 | 0,50 | 0,50 |
| 1,6-Hexandiol | 6,00 | 6,00 | 3,00 | 6,00 | - |
| Talkum | 0,50 | 0,50 | 0,50 | 0,50 | 0,50 |
| Methylparaben | 0,20 | 0,20 | 0,20 | 0,20 | 0,20 |
| Glycerin | 4,50 | 4,50 | 3,00 | 4,50 | 3,00 |
| Dry Flo Plus | 1,00 | 1,00 | 1,00 | 1,00 | 1,00 |
| Natipide 2 PG | 1,00 | 1,00 | 1,00 | 1,00 | 1,00 |
| Phenoxyethanol | 0,40 | 0,40 | 0,40 | 0,40 | 0,40 |
| Maltosin | 0,1 | 0,2 | 0,5 | 1,0 | 2,0 |
| Coffein | 0,50 | 1,00 | - | - | 0,35 |
| Carnitin | - | 0,10 | 0,50 | - | - |
| Matrixyl 3000 | 1,00 | - | - | 4,00 | 2,00 |
| Ridulisse C | - | - | 3,00 | - | 0,50 |
| Parfum | 0,40 | 0,40 | 0,40 | 0,40 | 0,40 |
| Aqua | ad 100 | ad 100 | ad 100 | ad 100 | ad 100 |

### 5. Wasser-in-Öl-Emulsion

5.1 - 5.3 sind bevorzugte Ausführungsformen von Cremes mit haarwuchsinhibierendem Effekt auf der Basis einer reichhaltigen Wasser-in-Öl-Emulsion, die in die Haut einmassiert werden kann.

| | 5.1 | 5.2 | 5.3 |
|---|---|---|---|
| Lameform TGI | 3,00 | 3,00 | 3,00 |
| Elfacos ST 37 | 0,50 | 0,50 | 0,50 |
| Microcrystalline Wax | 3,00 | 3,00 | 3,00 |
| Softisan 649 | 1,00 | 1,00 | 1,00 |
| Paraffinöl | 8,00 | 8,00 | 8,00 |
| Vaseline | 2,00 | 2,00 | 2,00 |
| Vitamin E Acetat | 2,00 | 2,00 | 2,00 |
| Methylparaben | 0,30 | 0,30 | 0,30 |
| Propylparaben | 0,30 | 0,30 | 0,30 |
| Isopropylisostearat | 8,00 | 8,00 | 8,00 |
| 1,3-Butylenglycol | 5,00 | 6,00 | 7,00 |
| Maltosin | 0,1 | 0,2 | 0,5 |
| Coffein | 0,50 | 0,50 | - |
| Carnitin | - | 0,10 | 0,50 |
| Milchsäure 80%ig | 0,60 | 0,60 | 0,60 |
| Panthenol | 0,5 | 1,0 | 0,5 |
| Tephrosia pupurea Seed Extract | 0,1 | - | - |
| Hydrovance | 2,00 | 4,00 | - |
| Parfum | 0,2 | 0,2 | 0,2 |
| Water | ad 100 | ad 100 | ad 100 |

### 6. Gesichtswasser

6.1 - 6.3 sind bevorzugte Ausführungsformen von Gesichtswässern mit haarwuchsinhibierendem Effekt.

| | 6.1 | 6.2 | 6.3 |
|---|---|---|---|
| Dipropylenglycol | 10,00 | 10,00 | 10,00 |
| Chlorhexidindigluconat | 1,00 | 1,00 | 1,00 |
| Synperonic PE7 L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil / Trideceth 9 / Propylene Glycol | 0,50 | 0,50 | 0,50 |
| Caomint | 0,50 | 0,50 | 0,50 |
| Maltosin | 0,1 | 0,2 | 0,5 |
| Coffein | 0,20 | - | - |
| Ridulisse C | 1,00 | - | - |
| Fucogel 1000 | - | - | 1,00 |
| Betain | - | 1,00 | - |
| Parfum | 0,20 | 0,20 | 0,20 |
| Aqua | ad 100 | ad 100 | ad 100 |

### 7. Wasser-in-Silicon-Emulsionen

7.1 - 7.3 sind bevorzugte Ausführungsformen von Cremes mit einem haarwuchsinhibierenden Effekt, auf der Basis einer reichhaltigen und dennoch nicht-fettenden Wasser-in-Silicon-Emulsion, die in die Haut einmassiert werden kann.

| | 7.1 | 7.2 | 7.3 |
|---|---|---|---|
| Dimethicone (100 cSt) | 2,50 | 2,50 | 2,50 |
| Cyclomethicone | 25,00 | 25,00 | 25,00 |
| Abil EM 90 | 2,00 | 2,00 | 2,00 |
| Natriumchlorid | 2,00 | 2,00 | 2,00 |
| Symdiol 68 | 0,30 | 0,30 | 0,30 |
| Phenoxyethanol | 0,40 | 0,40 | 0,40 |
| Maltosin | 0,1 | 0,2 | 0,5 |
| Coffein | - | 0,20 | - |
| Ridulisse C | - | 2,00 | 1,00 |
| Rhamnosoft | 2,00 | - | - |
| Hydrovance | - | 3,00 | - |
| Folsäure | - | - | 0,10 |
| Parfum | 0,30 | 0,30 | 0,30 |
| Aqua | ad 100 | ad 100 | ad 100 |

### 8. Beispielserie:

Die Zusammensetzungen 8.1 - 8.4 sind bevorzugte Ausführungsformen von Tagescremes (Öl-in-Wasser-Emulsionen) für besonders anspruchsvolle Haut mit haarwuchsinhibierendem Effekt.

| | 8.1 | 8.2 | 8.3 | 8.4 |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Dimethicone (350 cSt) | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glyceryl | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| Algenextrakt | 1,00 | - | - | - |
| Symdiol 68 | 0,30 | 0,50 | - | - |
| DSH-CN | 5,00 | 5,00 | 5,00 | 5,00 |
| Hydrovance | 4,30 | 2,50 | 8,60 | 10,00 |
| Maltosin | 1,5 | 0,2 | 0,5 | 1,0 |
| Coffein | - | 0,30 | - | 0,10 |
| Natrulon RC 50 DG | - | - | 1,00 | - |
| Phytokine | 2,00 | | - | - |
| Matrixyl 3000 | - | 2,00 | - | - |
| Ridulisse C | - | - | 2,00 | 2,50 |
| Argireline | 1,00 | - | - | - |
| Sepilift DPHP | - | 0,10 | - | - |
| Modulene | - | - | 1,00 | - |
| Collaxyl | - | - | - | 2,00 |
| Ederline H | 2,00 | - | - | - |
| Perfume | 0,10 | 0,10 | 0,10 | 0,10 |
| Aqua | ad 100 | ad 100 | ad 100 | ad 100 |

### 9. Hautcremes:

Die Zusammensetzungen 9.1 - 9.3 sind weitere bevorzugte Ausführungsformen von Cremes (Öl-in-Wasser-Emulsionen) mit haarwuchsinhibierendem Effekt für besonders anspruchsvolle Haut.

| | 9.1 | 9.2 | 9.3 |
|---|---|---|---|
| Cetearyl Isononanoate | 4,00 | 4,00 | 4,00 |
| Mineral oil | 6,00 | 6,00 | 6,00 |
| Cutina CBS | 2,00 | 2,00 | 2,00 |
| Cutina FS 45 | 1,50 | 1,50 | 1,50 |
| Eumulgin B3 | 1,00 | 1,00 | 1,00 |
| Dimethicone (350 cSt) | 1,00 | 1,00 | 1,00 |
| Tocopherylacetat | 0,50 | 0,50 | 0,50 |
| Propylparaben | 0,30 | 0,30 | 0,30 |
| Pemulen TR 1 | 0,27 | 0,27 | 0,27 |
| 1,3-Butylenglycol | 5,00 | 3,00 | 3,00 |
| Milchsäure 80 % | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Pantolacton | 0,50 | - | - |
| Seanergilium BG | - | 1,00 | - |
| Natriumchlorid | 0,05 | 0,05 | 0,05 |
| Sepigel 305 | 0,50 | 0,50 | 0,50 |
| Calmosensine | 1,00 | 2,00 | - |
| Keltrol SF | 0,20 | 0,20 | 0,20 |
| Hydrovance | 4,30 | 2,50 | 8,60 |
| Natriumascorbylphosphat | 1,00 | - | - |
| Keratec Pep | - | 2,00 | - |
| Kreatin | - | - | 0,10 |
| Retinol | 0,10 | - | - |
| Maltosin | 2,0 | 0,5 | 1,5 |
| Coffein | 0,20 | - | - |
| Perfume | 0,30 | 0,30 | 0,30 |
| Aqua | ad 100 | ad 100 | ad 100 |

### 10. Hautcremes auf Basis einer Lipoprotein-Creme (Öl-in-Wasser-Emulsion mit Proteinemulgator):

Die Zusammensetzungen 10.1 - 10.6 sind bevorzugte Ausführungsformen von Cremes mit haarwuchsinhibierendem Effekt auf Basis einer besonders milden Emulsionsbasis mit einem natürlichen Protein-Emulgator (Hibiscin® HP LS 9198), mit besonders milder Wirkung insbesondere für frisch rasierte Haut.

| | 10.1 | 10.2 | 10.3 | 10.4 | 10.5 | 10.6 |
|---|---|---|---|---|---|---|
| Myritol® PC | 3,5 | 3,5 | 3,5 | 3,5 | 3,5 | 3,5 |
| Lanette® 22 | 3,0 | 3,0 | 3,0 | 3,0 | 3,0 | 3,0 |
| Cutina® GMS-V | 3,0 | 3,0 | 3,0 | 3,0 | 3,0 | 3,0 |
| Stenol® 16/18 | 2,0 | 2,0 | 2,0 | 2,0 | 2,0 | 2,0 |
| Distelöl | 3,0 | 3,0 | 3,0 | 3,0 | 3,0 | 3,0 |
| Controx® KS | 0,05 | 0,05 | 0,05 | 0,05 | 0,05 | 0,05 |
| Dimethicone (350 cSt) | 1,0 | 1,0 | 1,0 | 1,0 | 1,0 | 1,0 |
| Isopropylstearat | 6,0 | 6,0 | 6,0 | 6,0 | 6,0 | 6,0 |
| Propylparaben | 0,2 | 0,2 | 0,2 | 0,2 | 0,2 | 0,2 |
| 1,3-Butylenglycol | 5,0 | 5,0 | 5,0 | 5,0 | 5,0 | 5,0 |
| 1,6-Hexandiol | 5,0 | 5,0 | 5,0 | 5,0 | 5,0 | 5,0 |
| Methylparaben | 0,2 | 0,2 | 0,2 | 0,2 | 0,2 | 0,2 |
| Hibiscin® HP LS 9198 | 3,0 | 3,0 | 3,0 | 3,0 | 3,0 | 3,0 |
| Citronensäure | 0,1 | 0,1 | 0,1 | 0,1 | 0,1 | 0,1 |
| Simulgel® NS | 2,0 | 2,0 | 2,0 | 2,0 | 2,0 | 2,0 |
| Pearl Protein Extract | - | - , | 2,00 | - | - | 1,00 |
| Maltosin | 0,1 | 0,2 | 0,5 | 1,0 | 1,5 | 0,1 |
| Coffein | 0,30 | 0,30 | 0,30 | - | - | - |
| Ridulisse C | 1,00 | 0,50 | 1,00 | - | - | - |
| Carnitin | 0,20 | - | - | 0,50 | 0,30 | 0,30 |
| Matrixyl 3000 | 3,00 | - | - | - | 2,00 | |
| Aqua | ad 100 | ad 100 | ad 100 | ad 100 | ad 100 | ad 100 |

### 11. Reinigungsgele mit haarwuchsinhibierendem Effekt:

Die Zusammensetzungen 11.1 - 11.3 sind bevorzugte Ausführungsformen von Reinigungsgelen mit haarwuchsinhibierendem Effekt.

| | 11.1 | 11.2 | 11.3 |
|---|---|---|---|
| Carbomer | 1,40 | 1,40 | 1,40 |
| 1,3-Butylenglycol | 4,00 | 4,00 | 4,00 |
| Sodium Benzoate | 0,40 | 0,40 | 0,40 |
| Plantaren® 1200 | 7,50 | 7,50 | 7,50 |
| Dehyton® K | 3,40 | 3,40 | 3,40 |
| Texapon® SB 3 | 5,00 | 5,00 | 5,00 |
| Cetiol® HE | 0,50 | 0,50 | 0,50 |
| Lamesoft® PO 65 | 5,00 | 5,00 | 5,00 |
| Controx KS | 0,05 | 0,05 | 0,05 |
| Sodium pyrrolidone carboxylic acid | 1,60 | 1,60 | - |
| Pantolactone | 1,00 | 1,00 | - |
| Tetrasodium EDTA | 0,25 | 0,25 | 0,25 |
| Sodium Lactate | 1,80 | - | - |
| Panthenol | 0,50 | 0,50 | 0,50 |
| Maltosin | 0,1 | 0,2 | 0,5 |
| Coffein | 0,30 | 0,30 | 0,30 |
| Ridulisse C | 1,00 | - | - |
| Carnitin | 0,20 | - | 0,20 |
| Perfume | 0,40 | 0,40 | 0,40 |
| Aqua | ad 100 | ad 100 | ad 100 |

### 12. Zweiphasen-Anti-Haarwuchs-Massagefluid für die Körperhaut

Die Zusammensetzungen 12.1 und 12.2 sind bevorzugte Ausführungsformen von Massagefluiden mit haarwuchsinhibierendem Effekt auf Basis einer Zweiphasenzusammensetzung, die direkt vor dem Gebrauch durch Schütteln in eine Emulsion überführt wird und sich besonders leicht in die Haut einmassieren lässt. Die Zusammensetzung ruft ein angenehm erfrischendes Hautgefühl hervor.

| | 12.1 | 12.2 |
|---|---|---|
| Ocaline | 1,000000 | 1,000000 |
| Milchsäure 80% DAB | 0,100000 | 0,100000 |
| Dinatriumphosphat wasserfrei | 0,100000 | 0,100000 |
| D-Panthenol 75 % | 0,100000 | 0,100000 |
| Parfum | 0,150000 | 0,150000 |
| Brij 30 | 0,500000 | 0,500000 |
| Trisiloxane Fluid 1 cs | 20,000000 | 20,000000 |
| Maltosin | 0,100000 | 0,200000 |
| Coffein | 0,500000 | - |
| Theobromin | | 0,300000 |
| Ridulisse C | 1,000000 | - |
| Carnitin | 0,200000 | - |
| Ethanol 96 % | 10,000000 | 10,000000 |
| Wasser, vollentsalzt | ad 100,000000 | ad 100,000000 |

### 13. Beispiele für Zusammensetzungen zum Tränken von Tüchern

| | Bestandteile | 13.1 | 13.2 | 13.3 | 13.4 |
|---|---|---|---|---|---|
| Phase 1 | PEG-40 Hydrogenated Castor Oil | 0,1 | 0,1 | 0,1 | - |
| | Trideceth-9 | 0,1 | 0,1 | 0,1 | - |
| | Parfum | 0,3 | 0,1 | 0,1 | 0,1 |
| Phase 2 | Hexyllaurat | 2,0 | - | - | - |
| | Dicaprylylcarbonat | - | - | 2,0 | - |
| | Dipropylenglycol | - | 2,0 | - | - |
| Phase 3 | Allantoin | 0,5 | - | - | - |
| | Citronensäure (Monohydrat) | 1,5 | 1,5 | 1,5 | 1,5 |
| | Trinatriumcitrat (Dihydrat) | 2,0 | 2,0 | 2,0 | 2,0 |
| | Decylglucosid | - | - | - | 2,0 |
| | Pemulen TR 1 | - | - | - | 0,2 |
| | Maltosin | 0,1 | 0,2 | 0,5 | 1,0 |
| | Coffein | 0,30 | 0,30 | 0,30 | - |
| | Ridulisse C | 1,00 | 0,50 | 1,00 | - |
| | Carnitin | 0,20 | - | - | 0,50 |
| | Wasser | ad 100 | ad 100 | ad 100 | ad 100 |

Mit diesen Tränkzusammensetzungen wurden verschiedene Viskose-Vliesstoffe ausgerüstet. Es wurden gelochte, ungelochte, genoppte, einlagige, zweilagige und dreilagige Vliese ausgerüstet. Die Tücher wurden sowohl als feuchte Tücher als auch als trockene Tücher (das heißt, nach dem Ausrüsten wurden die Tücher bis auf einen Restwassergehalt kleiner 10 Gew.-%, bevorzugt kleiner 5 Gew.-%, getrocknet, um direkt vor Gebrauch mit einer kleinen Menge an Wasser wieder befeuchtet zu werden oder auf feuchter Haut angewendet zu werden) verwendet. Als besonders bevorzugt erwiesen sich genoppte Tücher (beispielsweise mit heiß aufgesprühten Kunststoffnoppen) und Tücher mit grober Oberflächenstruktur, da diese den gewünschten Massageeffekt besonders vorteilhaft unterstützen. Derartige Tücher sind besonders vorteilhaft zur Anwendung unterwegs geeignet.

### 14. Matrixpflaster mit haarwuchsinhibierendem Effekt

| | 14.1 | 14.2 | 14.3 | 14.4 | 14.5 |
|---|---|---|---|---|---|
| DURO-TAK® 387-2516 | 76 | 76 | 76 | 76 | 76 |
| Aloe Vera Gel | 1 | - | 1 | - | - |
| Propylenglycolmonooleat | 5 | 5 | - | - | - |
| Tween®-80 | - | - | 5 | 3 | 5 |
| Natriumcitrat | 0,1 | 0,1 | 0,1 | 0,1 | 0,1 |
| Controx® KS | 0,05 | 0,05 | 0,05 | 0,05 | 0,05 |
| Maltosin | 0,1 | 0,2 | 0,5 | 1,0 | 1,5 |
| Coffein | 0,30 | 0,30 | 0,30 | - | - |
| Ridulisse C | 1,00 | 0,50 | 1,00 | - | - |
| Carnitin | 0,20 | - | - | 0,50 | 0,30 |
| Matrixyl 3000 | 3,00 | - | - | - | 2,00 |
| Wasser | ad 100 | ad 100 | ad 100 | ad 100 | ad 100 |

### 15. Reservoirpflaster mit haarwuchsinhibierendem Effekt

| Bestandteile des Wirkstoffreservoirs | 15.1 | 15.2 |
|---|---|---|
| Maltosin | 1,0 | 0,25 |
| Coffein | 0,30 | 0,30 |
| Ridulisse C | - | 0,50 |
| Carnitin | 0,50 | - |
| Ederline H | 1,0 | 1,0 |
| Carbopol® 980 | 0,5 | - |
| Pemulen®TR 1 | - | 0,5 |
| NaOH | 0,1 | 0,1 |
| Titandioxid | 0,2 | 0,2 |
| Ethanol | 1,0 | 1,0 |
| Polyvinylalkohol | 2,0 | 1,0 |
| Carboxymethylcellulose | 1,0 | 0,5 |
| Glycerin | 10 | 20 |
| Sorbitol | 7,0 | 5,0 |
| 1,3-Butandiol | 3,0 | 3,0 |
| Tween®-80 | 0,05 | 0,05 |
| Paraffinöl | 5,0 | 2,0 |
| Natriumcitrat | 0,1 | 0,1 |
| Controx KS | 0,05 | 0,05 |
| Wasser | ad 100 | ad 100 |

Die Bestandteile der Wirkstoffreservoir-Rezepturen 15.1 bzw. 15.2 wurden miteinander vermischt, so dass eine gelartige Masse entstand. Mit dieser Masse wurden Pflasterträger beschichtet, so dass sogenannte Reservoirpflaster erhalten wurden. Diese Pflaster werden sowohl auf die trockene Haut als auch auf die angefeuchtete Haut gelegt und verbleiben dort für eine Zeit von wenigen Sekunden bis einigen Stunden.

### 16. Körpercremes

Die nachfolgenden Rezepturen 16.1 und 16.2 sind Beispiele für erfindungsgemäße Anti-Haarwuchs-Körpercremes. Diese werden bevorzugt in die Haut der Unterschenkel und der Oberschenkel einmassiert.

| | 16.1 | 16.2 |
|---|---|---|
| Paraffinum Liquidum | 20,00 | 20,00 |
| Arlacel165 | 4,00 | 4,00 |
| Octyldodecanol | 1,00 | 1,00 |
| Cetearyl Isononanoate | 1,50 | 1,50 |
| Cremophor A 6 | 2,00 | 2,00 |
| Behenylalkohol | 1,60 | 1,60 |
| Silikonöl 350 cs | 3,00 | 3,00 |
| Brij 721 VP | 0,40 | 0,40 |
| Propylparaben | 0,20 | 0,20 |
| Controx KS | 0,25 | 0,25 |
| Propandiol-1,2 | 3,00 | 3,00 |
| Glycerin 86% pflanzlich | 5,00 | 5,00 |
| Polyethylenglykol MG 400 | 1,70 | 1,70 |
| Methylparaben | 0,20 | 0,20 |
| Phenoxyethanol, rein | 0,50 | 0,50 |
| Carbopol ETD 2020 | 0,50 | 0,50 |
| Natriumhydroxid Perlen | 0,03 | 0,03 |
| Citronensäure Monohydrat | 0,10 | 0,10 |
| Trinatriumcitrat Dihydrat | 0,10 | 0,10 |
| Maltosin | 0,5 | 1,0 |
| Coffein | 0,50 | 0,50 |
| Carnitin | 0,20 | 0,20 |
| Herbasol Extrakt Capsicum | - | 0,50 |
| Parfum | 0,30 | 0,20 |
| Simulgel NS | 1,00 | 1,10 |
| Wasser | ad 100,00 | ad 100,00 |

**Liste der verwendeten Rohstoffe**

| Handelsname | INCI-Bezeichnung | Lieferant/Hersteller |
|---|---|---|
| Abil EM 90 | Cetyl PEG/PPG-10/1 Dimethicone | Degussa |
| Argireline | Acetyl-Hexapeptide-3 | Lipotec |
| Arlacel 165 | Glyceryl Stearate, PEG-100 Stearate | Uniqema |
| Brij 30 | Laureth-4 | Uniqema |
| Brij 721 VP | STEARETH-21 | Uniqema |
| Calmosensine | BUTYLENE GLYCOL, WATER, LAURETH-3, HYDROXYETHYLCELLULOSE, Acetyl Dipeptide-1 Cetylester | Sederma |
| Caomint | Propylene Glycol, Aqua, Mentha piperita, Theobroma cacao | Solabia |
| Carbopol 980 | Carbomer | Noveon |
| Carbopol ETD 2020 | Acrylates/C10-30 Alkyl Acrylate Crosspolymer | Noveon |
| Cetiol® HE | PEG-7 Glyceryl Cocoate | Cognis |
| Cetiol® SB 45 | Butyrospermum parkii (Shea butter) | Cognis |
| Controx KS | Tocopherol, Hydrogenated Palm Glycerides Citrate | Cognis |
| Cremophor A 6 | CETEARETH-6, STEARYL ALCOHOL | BASF |
| Cutina CBS | Glyceryl Stearate / Cetearyl Alcohol / Cetyl Palmitate / Cocoglycerides im Gewichtsverhältnis 7:1:1:1 | Cognis |
| Cutina GMS-V | Glyceryl Stearate (Mischung aus Glycerylmono- und distearat) | Cognis |
| Cutina MD | Glyceryl Stearate (Mischung aus Glycerylmono- und distearat) | Cognis |
| Deliner | Zea Mays (Corn) Kernel Extract, Butylene Glycol, Xanthan Gum | Coletica |
| Dow Corning 1501 Fluid | Cyclomethicone (85 - 86 Gew.-%), Dimethiconol (14-15 Gew.-%) | Dow Corning |
| Dow Corning 9040 | Cyclomethicone/ Dimethicone Crosspolymer (87-88%/12-13 %) | Dow Corning |
| Dry Flo Plus | Aluminium Starch Octenylsuccinate | National Starch |
| DSH CN | Water, Dimethylsilanol Hyaluronate | Exsymol |
| DURO-TAK® 387-2516 | Polyacrylate, Ethylcellulose, Polyvinylpyrrolidon | National Starch and Chemical |
| Ederline H | PEG-40 Hydrogenated Castor Oil, PPG-2-Ceteareth-9, Pyrus Malus (Apple) Fruit Extract | Seporga |
| Elfacos ST 37 | PEG-45/Dodecyl Glycol Copolymer | Akzo Nobel |
| Eumulgin B 3 | Ceteareth-30 | Cognis |
| Fucogel 1000 | Biosaccharide Gum-1 | Solabia |
| Glistin | Water, L-Glutamylaminoethyl indole | Exsymol |
| Herbasol Extrakt Capsicum | Propylene Glycol, Aqua (Water), Capsicum Frutescens Fruit Extract,' Sorbitol | Cosmetochem |
| Hibiscin® HP-LS-9198 | Water, Hibiscus esculentus Seed Extract, Phenoxyethanol | Laboratoires Sérobiologiques |
| Hydagen CMF | Chitosan Glycolate | Cognis |
| Hydrovance | Wasser, Bis-N,N'-(2-Hydroxyethyl)harnstoff, Harnstoff, Ammoniumlactat | National Starch |
| Keltrol SF | Xanthan Gum | Kelco |
| Keratec Pep | Water, Hydrolyzed Keratin | Croda |
| Kombuchka | Saccharomyces/Xylinum/Black Tea Ferment, Glycerin, Hydroxyethylcellulose | Sederma |
| Lameform TGI | Polyglyceryl-3 Diisostearate | Coqnis |
| Lamesoft® PO 65 | Coco-Glucoside, Glyceryl Oleate, Water | Coqnis |
| Lanette® 22 | Behenyl Alcohol | Cognis |
| Liftiline | Aqua, Hydrolyzed Wheat Protein (7 - 10 Gew.-% Aktivsubstanz) | Silab |
| Lipoid S 75-3 | Aqua, Lecithine | Lipoid GmbH |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alkohol, Cetearyl Glucoside | Seppic |
| Myritol 318 | Caprylic/Capric Triglyceride | Cognis |
| Myritol® PC | PROPYLENE GLYCOL DICAPRYLATE/DICAPRATE | Cognis |
| Natipide 2 PG | Aqua, Propylene Glycol, Lecithin | Phospholipid GmbH |
| Natrulon RC 50 DG | WATER, CARNITINE, POLYGLYCERIN-10 | Lonza |
| Neo Heliopan AP | Disodium Phenyl Dibenzimidazole Tetrasulfonate | Symrise |
| Novata AB | Cocoglycerides | Cognis |
| Ocaline | Sea Water (and) Water (and) Cucurbita pepo (pumpkin) seed extract | Soliance |
| Olea europ. Fol extr. S. sicc. | Olea Europea (Olive) Leaf Extract | Fruitarom |
| Pearl Protein Extract | Aqua, Hydrolyzed Conchiolin Protein | Maruzen |
| Pemulen TR 1 | Acrylates/C10-30 Alkyl Acrylate Crosspolymer | Noveon |
| Photosomes | Aqua, Lecithine, Plancton Extract | AGI Dermatics |
| Phytokine | Aqua, Butylene Glycol, Hydrolyzed Soy Protein, Methylparaben, Ethylparaben, Propylparaben (0,4 - 0,8 Gew.-% Aktivsubstanz) | Coletica |
| Plantaren® 1200 | Lauryl Glucoside, ca. 50 % Aktivsubstanz | Cognis |
| Rhamnosoft | Biosaccharide Gum-2 | Solabia |
| Ridulisse C | Water, HYDROLYZED SOY PROTEIN (3 - 5 Gew.-% Aktivsubstanz) | Silab |
| Seanergilium BG | Butylene Glicol, Laminaria Digitata Extract, Methylparaben | Coletica |
| Sepigel®305 | Aqua (Water) Polyacrylamide, C₁₃-C₁₄ Isoparaffin, Laureth-7 (Aktivsubstanz Verdickerpolymer ca. 45 - 49 Gew.-%) | SEPPIC |
| Sepilift DPHP | Dipalmitoyl Hydroxyproline | Seppic |
| Silymarin Phytosome | Silybum Marianum Extract and Phospholipids | Indena SpA |
| Simulgel NS | Aqua (Water)/Hydroxyethyl Acrylate / Sodium Acryloyldimethyl Taurate Copolymer / Squalane / Polysorbate 60 | Seppic |
| Softisan 649 | Bis-Diglyceryl Polyacyladipate-2 | Sasol |
| Stenol 16/18 | Cetearylalkohol | Cognis |
| Symdiol 68 | 1,2-Octandiol, 1,2-Hexandiol | Symrise |
| Synperonic PE L 64 | Poloxamer-184 | Uniqema |
| Tego Carbomer 140 | Carbomer | Degussa |
| Texapon® SB 3 | Aqua, Disodium Laureth Sulfosuccinate, (ca. 40 % Aktivsubstanz), Citric Acid | Cognis |
| Tween®-80 | Polysorbate-80 | |
| Uvinul MBC 95 | 4-METHYLBENZYLIDENE CAMPHOR | BASF |

## Patentansprüche

1. Nicht-therapeutische, kosmetische Verwendung einer kosmetischen oder dermatologischen Zusammensetzung, enthaltend in einem geeigneten kosmetischen oder dermatologischen Träger, der in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, eines Schaumes, eines Puders oder einer Mischung mit mindestens einem als medizinischen Klebstoff geeigneten Polymer vorliegt, mindestens eine Verbindung aus der Gruppe der Advanced Glycation Endproducts (AGEs) und/oder deren Vorläufern, ausgewählt aus den Reaktionsprodukten der Umsetzung mindestens einer Verbindung aus der Gruppe der Zucker, bevorzugt ausgewählt aus den Aldosen, vorzugsweise ausgewählt aus Glucose, Laktose, Galactose und/oder Mannose, mit mindestens einer Verbindung aus der Gruppe der Aminosäuren, bevorzugt ausgewählt aus Lysin, Hydroxylysin, Arginin, Tryptophan und/oder Histidin, **dadurch gekennzeichnet, dass** sie mindestens eine Verbindung gemäß Formel 15 oder 15a enthält: 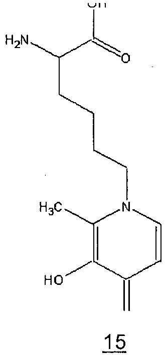 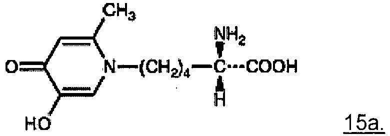 zur topischen Behandlung der Haut.

2. Verwendung nach Anspruch 1, **dadurch gekennzeichnet, dass** die Zusammensetzung - bezogen auf das Gewicht der anwendungsbereiten Zusammensetzung - 0,00001 bis 15 Gew.-%, vorzugsweise 0,0001 bis 10 Gew.-%, besonders bevorzugt 0,001 bis 5 Gew.-%, weiter bevorzugt 0,01 bis 4 Gew.-% und insbesondere 0,1 bis 2 Gew.-% Advanced Glycation Endproducts (AGEs) und/oder deren Vorläufer enthält.

3. Verwendung nach einem der Ansprüche 1 oder 2, **dadurch gekennzeichnet, dass** die Zusammensetzung - bezogen auf das Gewicht der anwendungsbereiten Zusammensetzung - 0,0001 bis 15 Gew.-%, vorzugsweise 0,001 bis 12,5 Gew.-%, besonders bevorzugt 0,01 bis 10 Gew.-%, weiter bevorzugt 0,1 bis 5 Gew.-% und insbesondere 0,25 bis 2,5 Gew.-% Maltosin (Formeln 15) enthält.

4. Verwendung gemäß einem der vorstehenden Ansprüche, **dadurch gekennzeichnet, dass** in der Zusammensetzung Purin und/oder mindestens ein Purin-Derivat enthalten ist, bevorzugt ausgewählt aus Verbindungen der allgemeinen Struktur (PUR-I), 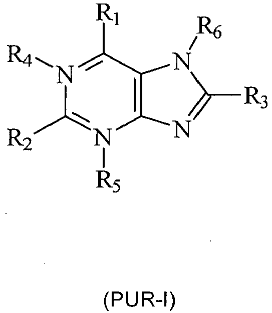 in der die Reste R¹, R² und R³ unabhängig voneinander ausgewählt sind aus -H, -OH, -NH₂, -SH und die Reste R⁴, R⁵ und R⁶ unabhängig voneinander ausgewählt sind aus -H, einem C₁- bis C₄-Alkylrest, einem C₁- bis C₄-Monohydroxyalkylrest, einem C₂- bis C₄-Polyhydroxyalkylrest, einem (C₁-bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe.

5. Verwendung gemäß Anspruch 4, **dadurch gekennzeichnet, dass** das Purin-Derivat ausgewählt ist aus Verbindungen der allgemeinen Struktur (PUR-II), 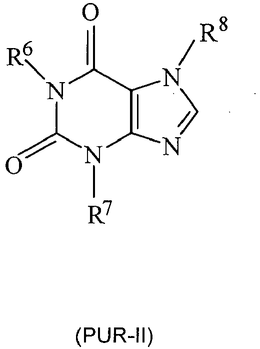 wobei in der Formel (PUR-II) die Reste R⁶, R⁷ und R⁸, die gleich oder verschieden sein können, ausgewählt sind aus einem Wasserstoffatom, einem C₁-C₄-Alkylrest, einem C₁-C₄-Monohydroxy-alkylrest, einem C₂-C₄-Polyhydroxyalkylrest, einem (C₁-C₄)-Alkoxy-(C₁-C₄)-alkylrest, einem C₁-C₄-Aminoalkylrest, einem Hydroxy-(C₁-C₄)-alkylaminorest, einem C₁-C₄-Hydroxyalkoxyrest, einem C₁-C₄-Hydroxyalkyl-(C₁-C₄)-aminoalkylrest oder einem (Di-C₁-C₄-Alkylamino)-(C₁-C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe.

6. Verwendung gemäß einem der vorstehenden Ansprüche, **dadurch gekennzeichnet, dass** das Purin-Derivat ausgewählt ist aus Verbindungen der allgemeinen Struktur (PUR-II), in denen R⁶ = R⁷= R⁸ = CH₃ (Coffein), oder R⁶ = H und R⁷= R⁸ = CH₃ (Theobromin) oder R⁸ = H und R⁶= R⁷ = CH₃ (Theophyllin) ist.

7. Verwendung gemäß einem der vorstehenden Ansprüche, **dadurch gekennzeichnet, dass** in der Zusammensetzung mindestens eine natürliche Betainverbindung enthalten ist, bevorzugt ausgewählt aus Betain (Me₃N⁺-CH₂-COO⁻) und Carnitin (Me₃N⁺-CH₂-CHOH-CH₂-COO⁻), jeweils mit Me = Methyl.

8. Verwendung gemäß einem der vorstehenden Ansprüche, **dadurch gekennzeichnet, dass** in der Zusammensetzung mindestens eine alkyl- oder hydroxyalkylsubstituierte Harnstoffverbindung enthalten ist, bevorzugt ausgewählt aus Verbindungen der allgemeinen Struktur (UREA-I), 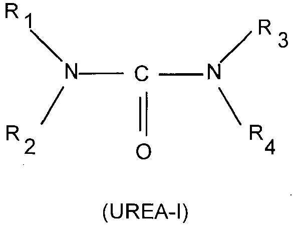 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ bis R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

9. Verwendung gemäß Anspruch 8, **dadurch gekennzeichnet, dass** die alkyl- oder hydroxyalkylsubstituierte Harnstoffverbindung Bis-N,N'-(2-Hydroxyethyl)harnstoff ist.

10. Verwendung gemäß einem der vorstehenden Ansprüche, **dadurch gekennzeichnet, dass** in der Zusammensetzung mindestens ein hyperämisierender Wirkstoff enthalten ist, bevorzugt ausgewählt aus
- Nicotinsäureestern, insbesondere Nicotinsäurebenzylester (Benzylnicotinat), Nicotinsäurebutoxyethylester (Nicoboxil), Nicotinsäuremethylester, Nicotinsäureethylester, Nicotinsäurehexylester, Nicotinsäureisopropylester, Nicotinsäuremyristylester, Tetrahydrofurfuryl-Nicotinat (Thurfyl-Nicotinat) und Tetrahydrofurfuryl-Nicotinat-Hydrochlorid,
- Pyridin-3-carbaldehyd (β-Pyridincarbaldehyd, Nicotinaldehyd),
- Salicylsäureestern, insbesondere Phenylsalicylat,
- Vanillylethern, insbesondere Vanillylbutylether,
- Carbonsäurevanillylamiden, insbesondere Nonivamid,
- Scharfstoffen, insbesondere Capsaicin, Cantharidin, Piperin, Gingerol, Zingeron, Myristicin, Safrol, Allicin, Sedamin, Sacculatal, Chalciporon, Isochalciporon, Velleral, Vellerol, und Isothiocyanaten (Senfölen), insbesondere Benzylsenföl,
- Extrakten aus Scharfstoff-Pflanzen, insbesondere aus Capsicum, insbesondere aus Capsicum annuum und Capsicum frutescens, aber auch aus Capsicum chinense, Capsicum baccatum und Capsicum pubescens, weiterhin aus Mauerpfeffer und aus der Zaunrübe,
- Terpentinöl,
- sowie Mischungen hiervon.

11. Verwendung gemäß einem der vorstehenden Ansprüche, **dadurch gekennzeichnet, dass** in der Zusammensetzung mindestens ein die Interaktion zwischen der extrazellulären Matrix und den Fibroblasten erhöhender und/oder verbessernder Wirkstoff enthalten ist.

12. Verwendung gemäß einem der vorstehenden Ansprüche, **dadurch gekennzeichnet, dass** in der Zusammensetzung mindestens ein weiterer Wirkstoff enthalten ist, ausgewählt aus
- Vitaminen, Provitaminen und Vitaminvorstufen der Gruppen B und E und den Estern der vorgenannten Substanzen,
- α-Hydroxycarbonsäuren, α-Ketocarbonsäuren, β-Hydroxycarbonsäuren und deren Ester-, Lacton- und/oder Salzform,
- Flavonoiden und Flavonoid-reichen Pflanzenextrakten,
- Ubichinon und Ubichinol sowie deren Derivaten,
- Silymarin,
- Ectoin,
- anorganischen und organischen UV-Filtersubstanzen,
- selbstbräunenden,
- hautberuhigenden sowie
- Mischungen dieser Wirkstoffe.

13. Verwendung gemäß einem der Ansprüche 1 - 12 zur Regulierung des Haarwuchses, insbesondere zur Verringerung des Haarwuchses.

## Claims

1. The non-therapeutic, cosmetic use of a cosmetic or dermatological composition, containing, in a suitable cosmetic or dermatological carrier, which is present in the form of a liquid, flowable or solid oil-in-water emulsion, water-in-oil emulsion, multiple emulsion, in particular an oil-in-water-in-oil emulsion or water-in-oil-in-water emulsion, macroemulsion, miniemulsion, microemulsion, PIT emulsion, nanoemulsion, Pickering emulsion, hydrodispersion, a hydrogel, a lipogel, a foam, a powder or a mixture having at least one polymer suitable as a medical adhesive, at least one compound from the group of advanced glycation end products (AGEs) and/or the precursors thereof, selected from the reaction products of the reaction of at least one compound from the group of sugar, preferably selected from the aldoses, preferably selected from glucose, lactose, galactose and/or mannose, having at least one compound from the group of amino acids, preferably selected from lysine, hydroxylysine, arginine, tryptophan and/or histidine, **characterized in that** said composition contains at least one compound according to formula 15 or 15a: 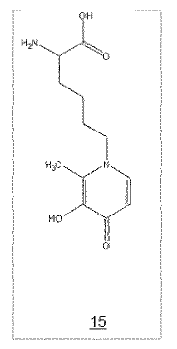 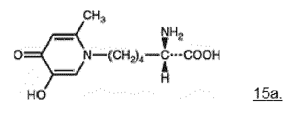 for the topical treatment of the skin.

2. The use according to claim 1, **characterized in that** the composition, based on the weight of the ready-to-use composition, contains from 0.00001 to 15 wt.%, preferably from 0.0001 to 10 wt.%, particularly preferably from 0.001 to 5 wt.%, more preferably from 0.01 to 4 wt.% and in particular from 0.1 to 2 wt.% of advanced glycation end products (AGEs) and/or the precursors thereof.

3. The use according to one of claims 1 or 2, **characterized in that** the composition, based on the weight of the ready-to-use composition, contains from 0.0001 to 15 wt.%, preferably from 0.001 to 12.5 wt.%, particularly preferably from 0.01 to 10 wt.%, more preferably from 0.1 to 5 wt.%, and in particular from 0.25 to 2.5 wt.% of maltosine (formula 15).

4. The use according to one of the preceding claims, **characterized in that** purine and/or at least one purine derivative is contained in the composition, preferably selected from compounds having the general structure (PUR-I), 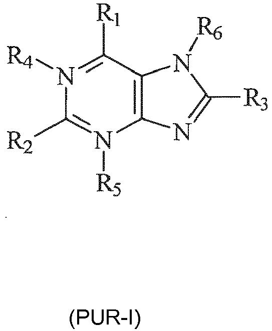 in which the functional groups R¹, R² and R³, independently of one another, are selected from -H, -OH, -NH₂, -SH, and the functional groups R⁴, R⁵ and R⁶, independently of one another, are selected from -H, a C₁ to C₄ alkyl functional group, a C₁ to C₄ monohydroxy alkyl functional group, a C₂ to C₄ polyhydroxy alkyl functional group, a (C₁ to C₄) alkoxy (C₁ to C₄) alkyl functional group, a C₁ to C₄ amino alkyl functional group, a hydroxy (C₁ to C₄) alkyl amino functional group, a C₁ to C₄ hydroxyalkoxy functional group, a C₁ to C₄ hydroxy alkyl (C₁ to C₄) amino alkyl functional group or a (Di-C₁ to C₄ alkyl amino) (C₁ to C₄) alkyl group, an optionally substituted aryl group, in particular a phenyl group, an optionally substituted heteroaryl group and an aryl C₁ to C₆ alkyl group.

5. The use according to claim 4, **characterized in that** the purine derivative is selected from compounds having the general structure (PUR-II), 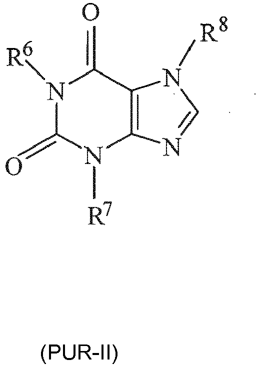 in which, in formula (PUR-II), the functional groups R⁶, R⁷ and R⁸, which can be identical or different, are selected from a hydrogen atom, a C₁-C₄ alkyl functional group, a C₁-C₄ monohydroxy alkyl functional group, a C₂-C₄ polyhydroxy alkyl functional group, a (C₁-C₄) alkoxy (C₁-C₄) alkyl functional group, a C₁-C₄ amino alkyl functional group, a hydroxy (C₁-C₄) alkyl amino functional group, a C₁-C₄ hydroxyalkoxy functional group, a C₁-C₄ hydroxy alkyl (C₁-C₄) amino alkyl functional group, or a (Di-C₁-C₄ alkyl amino) (C₁-C₄) alkyl functional group, an optionally substituted aryl group, in particular a phenyl group, an optionally substituted heteroaryl group and an aryl C₁-C₆ alkyl group.

6. The use according to one of the preceding claims, **characterized in that** the purine derivative is selected from compounds having the general structure (PUR-II), in which R⁶ = R⁷ = R⁸ = CH₃ (caffeine), or R⁶ = H and R⁷ = R⁸ = CH₃ (theobromine) or R⁸ = H and R⁶= R⁷ = CH₃ (theophylline).

7. The use according to one of the preceding claims, **characterized in that** at least one natural betaine compound is contained in the composition, preferably selected from betaine (Me₃N⁺-CH₂-COO⁻) and carnitine (Me₃N⁺-CH₂-CHOH-CH₂-COO⁻), in each case where Me = methyl.

8. The use according to one of the preceding claims, **characterized in that** at least one alkyl-substituted or hydroxyalkyl-substituted urea compound is contained in the composition, preferably selected from compounds having the general structure (UREA-I), 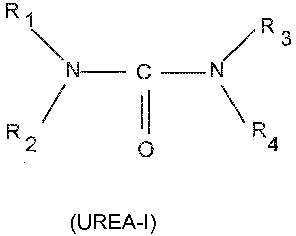 in which R₁, R₂, R₃ and R₄, independently of one another, represent a hydrogen atom, a methyl group, ethyl group, n-propyl group, isopropyl group, n-butyl group, secondary butyl group, tertiary butyl group, or C₂-C₆ hydroxyalkyl group which is substituted with from 1 to 5 hydroxyl groups or C₁-C₄ hydroxyalkyl groups, with the proviso that at least one of the functional groups R₁ to R₄ is a C₂-C₆ hydroxyalkyl group which is substituted with from 1 to 5 hydroxyl groups or C₁-C₄ hydroxyalkyl groups.

9. The use according to claim 8, **characterized in that** the alkyl-substituted or hydroxyalkyl substituted urea compound is Bis-N,N'-(2-hydroxyethyl) urea.

10. The use according to one of the preceding claims, **characterized in that** at least one hyperemic active ingredient is contained in the composition, preferably selected from
- nicotinic acid esters, in particular nicotinic acid benzyl ester (benzyl nicotinate), nicotinic acid butoxy ethyl ester (nicoboxil), nicotinic acid methyl ester, nicotinic acid ethyl ester, nicotinic acid hexyl ester, nicotinic acid isopropyl ester, nicotinic acid myristyl ester, tetrahydrofurfuryl nicotinate (thurfyl nicotinate) and tetrahydrofurfuryl nicotinate hydrochloride,
- pyridine-3-carbaldehyde (β-pyridinecarboxaldehyde, nicotinic aldehyde),
- salicylic acid esters, in particular phenyl salicylate,
- vanillyl ethers, in particular vanillyl butyl ether,
- carboxylic acid vanillyl amides, in particular nonivamide,
- pungent substances, in particular capsaicin, cantharidine, piperine, gingerol, zingerone, myristicin, safrole, allicin, sedamine, sacculatal, chalciporone, isochalciporone, velleral, vellerol, and isothiocyanates (mustard oils), in particular benzyl mustard oil,
- extracts from pungent plants, in particular from Capsicum, in particular from Capsicum annuum and Capsicum frutescens, but also from Capsicum chinense, Capsicum baccatum and Capsicum pubescens, furthermore from sedum and from bryony,
- turpentine oil,
- and mixtures thereof.

11. The use according to one of the preceding claims, **characterized in that** at least one active ingredient that increases and/or improves the interaction between the extracellular matrix and the fibroblasts is contained in the composition.

12. The use according to one of the preceding claims, **characterized in that** at least one additional active ingredient is contained in the composition, selected from
- vitamins, provitamins and vitamin precursors of the groups B and E and the esters of the aforementioned substances,
- α-hydroxy carboxylic acids, α-keto carboxylic acids, β-hydroxy carboxylic acids and the ester form, lactone form and/or salt form thereof,
- flavonoids and flavonoid-rich plant extracts,
- ubiquinone and ubiquinol and derivatives thereof,
- silymarin,
- ectoine,
- inorganic and organic UV-filter substances,
- self-tanning,
- skin-soothing and
- mixtures of these active ingredients.

13. The use according to any of claims 1-12 for regulating hair growth, in particular for reducing hair growth.

## Revendications

1. Utilisation cosmétique non thérapeutique d'une composition cosmétique ou dermatologique contenant, dans un support cosmétique ou dermatologique approprié, qui se présente sous la forme d'une émulsion huile-dans-eau liquide, fluide ou solide, d'une émulsion eau-dans-huile, d'une émulsion multiple, en particulier d'une émulsion huile-dans-eau-dans-huile ou eau-dans-huile-dans-eau, d'une macroémulsion, d'une miniémulsion, d'une microémulsion, d'une émulsion PIT, d'une nano-émulsion, d'une émulsion de Pickering, d'une hydrodispersion, d'un hydrogel, d'un lipogel, d'une mousse, d'une poudre ou d'un mélange comportant au moins un polymère approprié comme adhésif à usage médicale, au moins un composé du groupe des produits terminaux de glycation avancée (PTGA) et/ou de leurs précurseurs choisis parmi les produits réactionnels de la réaction d'au moins un composé du groupe des sucres, de préférence choisis parmi les aldoses, de préférence choisi parmi le glucose, le lactose, le galactose et/ou du le mannose, avec au moins un composé du groupe des acides aminés, de préférence choisis parmi la lysine, l'hydroxylysine, l'arginine, le tryptophane, et/ou l'histidine, **caractérisée en ce qu'**elle contient au moins un composé de la formule 15 ou 15a : 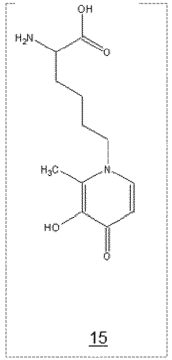 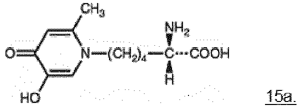 pour le traitement topique de la peau.

2. Utilisation selon la revendication 1, **caractérisé en ce que** la composition contient, par rapport au poids de la composition prête à l'emploi, de 0,00001 à 15% en poids, de préférence de 0,0001 à 10% en poids, de façon particulièrement préférée de 0,001 à 5% en poids, plus préférablement de 0,01 à 4% en poids et en particulier de 0,1 à 2% en poids de produits terminaux de glycation avancée (PTGA) et/ou de leurs précurseurs.

3. Utilisation selon l'une des revendications 1 ou 2, **caractérisée en ce que** la composition contient, par rapport au poids de la composition prête à l'emploi, de 0,0001 à 15% en poids, de préférence de 0,001 à de 12,5% en poids, de façon particulièrement préférée de 0,01 à 10% en poids, plus préférablement de 0,1 à 5% en poids et notamment de 0,25 à 2,5% en poids, de maltosine (formule 15).

4. Utilisation selon l'une des revendications précédentes, **caractérisée en ce que** la composition contient de la purine et/ou au moins un dérivé de la purine, choisie de préférence parmi les composés de la structure générale (PUR-I) 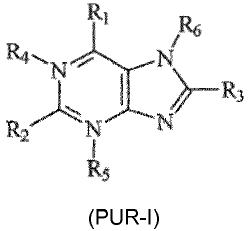 dans laquelle les radicaux R¹, R² et R³ sont choisis indépendamment parmi -H, -OH, -NH₂, -SH et les radicaux R⁴, R⁵ et R⁶ sont choisis indépendamment parmi -H, un radical alkyle en C₁ à C₄, un radical monohydroxyalkyle en C₁ à C₄, un radical polyhydroxyalkyle en C₂ à C₄, un radical (alcoxy en C₁ à C₄)-(alkyle en C₁ à C₄), un radical aminoalkyle en C₁ à C₄, un radical hydroxy-alkylamino en C₁ à C₄, un radical hydroxyalcoxy en C₁ à C₄, un radical (hydroxyalkyle en C₁ à C₄)-(aminoalkyle en C₁ à C₄) ou un radical (dialkylamino en C₁ à C₄)-alkyle en C₁ à C₄), un groupe aryle éventuellement substitué, en particulier un groupe phényle, un groupe hétéroaryle éventuellement substitué et un groupe aryl-alkyle en C₁ à C₆.

5. Utilisation selon la revendication 4, **caractérisée en ce que** le dérivé de la purine est choisi parmi les composés de la structure générale (PUR-II), 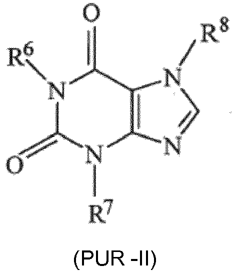 dans la formule (PUR -II), les radicaux R⁶, R⁷ et R⁸, qui peuvent être identiques ou différents, étant choisis parmi un atome d'hydrogène, un radical alkyle en C₁ à C₄, un monohydroxyalkyle en C₁ à C₄, un radical polyhydroxyalkyle en C₁ à C₄, un radical (alcoxy en C₁ à C₄)-(alkyle en C₁ à C₄), un radical aminoalkyle en C₁ à C₄, un radical hydroxy-alkylamino en C₁ à C₄, un radical hydroxyalcoxy en C₁ à C₄, un radical (hydroxyalkyl en C₁ à C₄)-(aminoalkyle en C₁ à C₄) ou un radical (di-alkylamino en C₁ à C₄)-(alkyle en C₁ à C₄), un groupe aryle éventuellement substitué, en particulier un groupe phényle, un groupe hétéroaryle éventuellement substitué, et un groupe aryle-alkyle en C₁ à C₆.

6. Utilisation selon l'une des revendications précédentes, **caractérisée en ce que** le dérivé de la purine est choisi parmi les composés de structure générale (PUR-II), dans lesquels R⁶ = R⁷ = R⁸ = CH₃ (caféine), ou R⁶ = H et R⁷ = R⁸ = CH₃ (théobromine), ou R⁸ = H et R⁶ = R⁷ = CH₃ (théophylline).

7. Utilisation selon l'une des revendications précédentes, **caractérisée en ce que** la composition contient au moins un composé de type bétaïne naturelle, de préférence choisi parmi la bétaïne (Me₃N⁺-CH₂-COO⁻), et la carnitine (Me₃N⁺-CH₂-CHOH-CH₂-COO⁻), avec à chaque fois Me = méthyle.

8. Utilisation selon l'une des revendications précédentes, **caractérisée en ce que** la composition contient au moins un un composé d'urée substitué par un alkyle ou un hydroxyalkyle, de préférence choisi parmi les composés de la structure générale (URÉE-I), 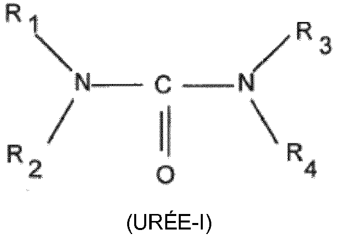 dans laquelle R₁, R₂, R₃ et R₄ représentent indépendamment un atome d'hydrogène, un groupe méthyle, éthyle, n-propyle, isopropyle, n-butyle, sec.-butyle, tert.-Butyle ou hydroxyalkyle en C₂ à C₆, qui est substitué par 1 à 5 groupes hydroxyle ou groupes hydroxyalkyle en C₁ à C₄, avec la condition qu'au moins un des radicaux R₁ à R₄ soit un groupe hydroxyalkyle en C₂ à C₆ qui est substitué par 1 à 5 groupes hydroxyle ou groupes hydroxyalkyle en C₁ à C₄.

9. Utilisation selon la revendication 8, **caractérisée en ce que** le composé d'urée substitué par un alkyle ou un hydroxyalkyle est le bis-N,N'-(2-hydroxyéthyl)urée.

10. Utilisation selon l'une des revendications précédentes, **caractérisée en ce que** la composition contient au moins une substance active hyperémiante, de préférence choisie parmi
- les esters d'acide nicotinique, en particulier l'ester benzylique de l'acide nicotinique (nicotinate de benzyle), le nicotinate de butoxyéthyle (Nicoboxil), le nicotinate de méthyle, le nicotinate d'éthyle, le nicotinate d'hexyle, le le nicotinate d'isopropyle, le nicotinate de myristyle, le nicotinate de tétrahydrofurfuryle (nicotinate de thurfyle) et l'hydrochlorure de nicotinate de tétrahydrofurfuryle,
- le pyridine-3-carbaldéhyde (β-pyridinecarbaldéhyde, nicotinaldéhyde)
- les esters d'acide salicylique, en particulier le salicylate de phényle,
- les éthers de vanillyle, en particulier le butyléther de vanillyle,
- les vanillylamides d'acides carboxyliques, en particulier la nonivamide,
- les substances piquantes, en particulier la capsaïcine, la cantharidine, la pipérine, le gingerol, la zingerone, la myristicine, le safrol, l'allicine, la sédamine, le sacculatal, le chalciporon, l'isochalciporon, le velleral, le vellerol et les isothio-cyanates (huiles de moutarde), en particulier la moutarde de benzyle,
- les extraits de plantes végétales à base de substances piquantes, notamment le Capsicum, en particulier le Capsicum annuum et le Capsicum frutescens, mais aussi le Capscum chinense, le Capsicum baccatum et le Capsicum pubescens, en outre de l'orpin et de la bryonia,
- la térébenthine,
- et leurs mélanges.

11. Utilisation selon l'une des revendications précédentes, **caractérisée en ce que** la composition contient au moins une substance active qui augmente et/ou améliore l'interaction entre la matrice extracellulaire et les fibroblastes.

12. Utilisation selon l'une des revendications précédentes, **caractérisée en ce que** la composition contient au moins une autre substance active choisie parmi
- les vitamines, les provitamines et les précurseurs de vitamines des groupes B et E et les esters des substances susmentionnées,
- les acides α-hydroxycarboxyliques, les acides α-cétocarboxyliques, les acides β-hydroxycarboxyliques et leur forme d'ester, de lactone et/ou de sel,
- les flavonoïdes et les extraits de plantes riches en flavonoïdes,
- l'ubiquinone et l'ubiquinol et leurs dérivés,
- la silymarine,
- l'ectoïne,
- les substances filtrantes UV minérales et organiques,
- les autobronzants,
- les apaisants cutanés et
- des mélanges de ces substances actives.

13. Utilisation selon l'une des revendications 1 à 12 pour la régulation la croissance des cheveux, en particulier pour réduire la croissance des cheveux.


### SEQUENCE LISTING

<110> Henkel AG & Co. KGaA
<120> Neue kosmetische Zusammensetzungen mit haarwuchsinhibierender Wirkung
<130> H 08208 PCT
<150> DE 102008059703.1
   <151> 2008-12-01
<160> 22
<170> PatentIn version 3.3
<210> 1
   <211> 4
   <212> PRT
   <213> Artificial
<220>
   <223> Artificial
<400> 1
   Gly Gln Pro Arg
   1
<210> 2
   <211> 4
   <212> PRT
   <213> Artificial
<220>
   <223> N-Palmitoyl
<400> 2
   Gly Gln Pro Arg
   1
<210> 3
   <211> 4
   <212> PRT
   <213> Artificial
<220>
   <223> Artificial
<400> 3
   Ala Gln Thr Arg
   1
<210> 4
   <211> 4
   <212> PRT
   <213> Artificial
<220>
   <223> tetrapeptide
<220>
   <221> misc_feature
   <222> (1)..(1)
   <223> Xaa chosen from Gly and amino acids that can replace Gly, preferably bAla, Ala, Val , Leu, Pro, Sar (Sarcosin), Ile.
<220>
   <221> misc_feature
   <222> (2)..(2)
   <223> Xaa chosen from Gln and amino acids that can replace Gln, preferably Asn, Lys, Orn, 5-Hydroxyprolin, Cirullin, Canavanin.
<220>
   <221> misc_feature
   <222> (4)..(4)
   <223> Xaa chosen from Pro or Arg and amino acids that can replace Pro or Arg, Arg replacing amino acids are preferably Pro, Lys His, Desmosin or Isodesmosin.
<400> 4
   Xaa Xaa Arg Xaa
   1
<210> 5
   <211> 4
   <212> PRT
   <213> Artificial
<220>
   <223> Artificial Sequence
<220>
   <221> misc_feature
   <222> (1)..(1)
   <223> Xaa chosen from Gly and amino acids that can replace Gly, preferably bAla, Ala, Val , Leu, Pro, Sar (Sarcosin), Ile.
<220>
   <221> misc_feature
   <222> (2)..(2)
   <223> Xaa chosen from Gln and amino acids that can replace Gln, preferably Asn, Lys, Orn, 5-Hydroxyprolin, Cirullin, Canavanin.
<220>
   <221> misc_feature
   <222> (3)..(3)
   <223> Xaa chosen from Pro or Arg and amino acids that can replace Pro or Arg, Arg replacing amino acids are preferably Pro, Lys His, Desmosin or Isodesmosin.
<400> 5
   Xaa Xaa Xaa Pro
   1
<210> 6
   <211> 4
   <212> PRT
   <213> Artificial
<220>
   <223> Artificial Sequence
<220>
   <221> misc_feature
   <222> (1)..(1)
   <223> Xaa chosen from Gly and amino acids that can replace Gly, preferably bAla, Ala, Val , Leu, Pro, Sar (Sarcosin), Ile.
<220>
   <221> misc_feature
   <222> (2)..(2)
   <223> Xaa chosen from Gln and amino acids that can replace Gln, preferably Asn, Lys, Orn, 5-Hydroxyprolin, Cirullin, Canavanin.
<220>
   <221> misc_feature
   <222> (4)..(4)
   <223> Xaa chosen from Pro or Arg and amino acids that can replace Pro or Arg, Arg replacing amino acids are preferably Pro, Lys His, Desmosin or Isodesmosin.
<400> 6
   Xaa Xaa Pro Xaa
   1
<210> 7
   <211> 4
   <212> PRT
   <213> Artificial
<220>
   <223> Artificial Sequence
<220>
   <221> misc_feature
   <222> (1)..(1)
   <223> Xaa chosen from Gly and amino acids that can replace Gly, preferably bAla, Ala, Val , Leu, Pro, Sar (Sarcosin), Ile.
<220>
   <221> misc_feature
   <222> (2)..(2)
   <223> Xaa chosen from Gln and amino acids that can replace Gln, preferably Asn, Lys, Orn, 5-Hydroxyprolin, Cirullin, Canavanin.
<400> 7
   Xaa Xaa Pro Arg
   1
<210> 8
   <211> 4
   <212> PRT
   <213> Artificial
<220>
   <223> Artificial Sequence
<220>
   <221> misc_feature
   <222> (1)..(1)
   <223> Xaa chosen from Gly and amino acids that can replace Gly, preferably bAla, Ala, Val , Leu, Pro, Sar (Sarcosin), Ile.
<220>
   <221> misc_feature
   <222> (2)..(2)
   <223> Xaa chosen from Gln and amino acids that can replace Gln, preferably Asn, Lys, Orn, 5-Hydroxyprolin, Cirullin, Canavanin.
<220>
   <221> misc_feature
   <222> (3)..(3)
   <223> Xaa chosen from Pro or Arg and amino acids that can replace Pro or Arg, Arg replacing amino acids are preferably Pro, Lys His, Desmosin or Isodesmosin.
<400> 8
   Xaa Xaa Xaa Arg
   1
<210> 9
   <211> 4
   <212> PRT
   <213> Artificial
<220>
   <223> Artificial Sequence
<400> 9
   Val Val Arg Pro
   1
<210> 10
   <211> 5
   <212> PRT
   <213> Artificial
<220>
   <223> preferably with N-Acyl or ester-bond
<400> 10
   Lys Thr Thr Lys Ser
   1 5
<210> 11
   <211> 5
   <212> PRT
   <213> Artificial
<220>
   <223> Artificial Sequence
<220>
   <221> MOD_RES
   <222> (1)..(1)
   <223> N-Palmitoyl-Lys
<400> 11
   Lys Thr Thr Lys Ser
   1 5
<210> 12
   <211> 5
   <212> PRT
   <213> Artificial
<220>
   <223> Artificial Sequence
<220>
   <221> MOD_RES
   <222> (1)..(1)
   <223> N-Palmitoyl-Tyr
<400> 12
   Tyr Gly Gly Phe Met
   1 5
<210> 13
   <211> 5
   <212> PRT
   <213> Artificial
<220>
   <223> Artificial Sequence
<400> 13
   Val Val Arg Pro Pro
   1 5
<210> 14
   <211> 5
   <212> PRT
   <213> Artificial
<220>
   <223> Artificial Sequence
<220>
   <221> MOD_RES
   <222> (1)..(1)
   <223> N-Palmitoyl-Tyr
<400> 14
   Tyr Gly Gly Phe Leu
   1 5
<210> 15
   <211> 5
   <212> PRT
   <213> Artificial
<220>
   <223> Artificial Sequence
<220>
   <221> MOD_RES
   <222> (1)..(1)
   <223> N-Benzyloxycarbonyl-Gly
<400> 15
   Gly Pro Phe Pro Leu
   1 5
<210> 16
   <211> 5
   <212> PRT
   <213> Artificial
<220>
   <223> Artificial Sequence
<400> 16
   Gly Pro Phe Pro Leu
   1 5
<210> 17
   <211> 6
   <212> PRT
   <213> Artificial
<220>
   <223> Artificial Sequence
<400> 17
   Val Gly Val Ala Pro Gly
   1               5
<210> 18
   <211> 6
   <212> PRT
   <213> Artificial
<220>
   <223> Artificial Sequence
<220>
   <221> MOD_RES
   <222> (1)..(1)
   <223> N-Palmitoyl-VAL
<400> 18
   Val Gly Val Ala Pro Gly
   1               5
<210> 19
   <211> 6
   <212> PRT
   <213> Artificial
<220>
   <223> Peptide 02 Vincience
<400> 19
   Ala Asp Leu Lys Pro Thr
   1               5
<210> 20
   <211> 6
   <212> PRT
   <213> Artificial
<220>
   <223> MOD_RES
<220>
   <221> MOD_RES
   <222> (4)..(4)
   <223> Xaa=Methylnorleucin
<220>
   <221> MOD_RES
   <222> (5)..(5)
   <223> Xaa=Homophenylalanin
<400> 20
   Ala Arg His Xaa Xaa Trp
   1 5
<210> 21
   <211> 6
   <212> PRT
   <213> Artificial
<220>
   <223> Hexapeptide-1
<400> 21
   Ala Arg His Leu Phe Trp
   1 5
<210> 22
   <211> 6
   <212> PRT
   <213> Artificial
<220>
   <223> Artificial Sequence
<400> 22
   Val Val Arg Pro Pro Pro
   1 5